(19) Europäisches Patentamt

European Patent Office

Office européen des brevets

(11) **EP 2 497 378 A2**

(12) **EUROPEAN PATENT APPLICATION**

(43) Date of publication:
**12.09.2012 Bulletin 2012/37**

(21) Application number: **12159268.7**

(22) Date of filing: **12.10.2006**

(51) Int Cl.:
*A23K 1/165* (2006.01)  *C12N 9/96* (2006.01)
*C12N 9/98* (2006.01)  *A61K 9/16* (2006.01)

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.10.2005 US 726494 P**

(62) Document number(s) of the earlier application(s) in accordance with Art. 76 EPC:
**06826039.7 / 1 940 241**

(71) Applicant: **Danisco US Inc.**
**Palo Alto, CA 94304-1013 (US)**

(72) Inventors:
• **Becker, Nathaniel T.**
**Hillsborough, CA California 94010 (US)**
• **Clarkson, Kathleen A.**
**Palo Alto, CA 94304-1013 (US)**
• **Dale, Douglas A.**
**Palo Alto, CA 94304-1013 (US)**

• **Fryksdale, Beth**
**San Jose, CA California 95118 (US)**
• **Gerbert, Mark S.**
**Pacifica, CA California 94044 (US)**
• **Partsuf, Michael**
**Palo Alto, CA California 94304 (US)**
• **Gravesen, Troels**
**DK-8210 Aarhus V (DK)**

(74) Representative: **HOFFMANN EITLE**
**Patent- und Rechtsanwälte**
**Arabellastrasse 4**
**81925 München (DE)**

Remarks:
•This application was filed on 13-03-2012 as a divisional application to the application mentioned under INID code 62.
•Claims filed after the date of filing of the application/receipt of the divisional application (Rule 68(4) EPC).

(54) **Stable, durable granules with active agents**

(57)  A stable, durable granule for feed compositions has a core, at least one active agent; and at least one coating. The active agent of the granule retains at least 50% activity, at least 60% activity, at least 70% activity, at least 80% activity after conditions selected from one or more of a) a feed pelleting process, b) a steam-heated feed pretreatment process, c) storage, d) storage as an ingredient in an unpelleted mixture, and e) storage as an ingredient in a feed base mix or a feed premixcomprising at least one compound selected from trace minerals, organic acids, reducing sugars, vitamins, choline chloride, and compounds which result in an acidic or a basic feed base mix or feed premix.

EP 2 497 378 A2

## Description

RELATED APPLICATIONS

This application claims priority under 35 U.S.C. §119 to U.S. Provisional Application Serial No. 60/726,494, filed October 12, 2005.

FIELD OF THE INVENTION

The present invention relates to stable, durable granules with active agents. Specifically, the invention relates to thermostable, durable granules with active agents, the granules being particularly suitable for inclusion in steam treatment processes, including pelleting and tableting processes and steam processing of feed, without appreciable loss of active agent activity. The stable, durable granules have dissolution profiles suitable to release the active agent to provide efficacy for its intended purpose. The activity of the active agents is retained following storage in unpelleted mixtures and steam treatment.

BACKGROUND OF THE INVENTION

The use of active agents, such as enzymes, in animal feed is common. Enzymes are known to improve digestibility of feed, reduce antinutritional factors in feed, and improve animal productivity. It is known in the industry that acidic and basic feed ingredients as well as particular ingredients of animal feeds, including, but not limited to trace minerals, organic or inorganic acids or bases, reducing sugars, and hygroscopic substances, particularly choline chloride and sodium chloride, have an adverse effect upon active agents such as other vitamins, proteins, antimicrobials, prebiotics, probiotics and enzymes; and, it is also known that some feed production processes are detrimental to active agents.
A problem exists in the industry to provide protective formulations to make the active agents suitable for storage in unpelleted animal feed mixtures, such as base mixes and premixes, which may be acidic or basic and contain the ingredients that have an adverse effect upon the stability of the active agents. One mechanism for the adverse effect is said to be oxidation-reduction (redox) reactions that occur between oxidizing and reducing compounds in the premixes in the presence of water. A study reported in BASF Technical Bulletin NU0013 reports that two commercially available enzyme containing granules retained, respectively, 86% and 81% activity after storage for 3 weeks in a feed premix, and 55% and 33% activity, respectively, after storage in the feed premix for 6 weeks. Currently some manufacturers of enzymes for the feed industry recommend that enzymes be protected with barrier packaging if they are to be stored in premixes, or that they be stored separately from the premixes, or that they be stored in premixes for only a short period of time.
Additionally, many active agents used in food and feed are heat labile. Thermal stability of enzymes and their ability to survive heat processing steps in the manufacture of animal feed is a problem in the industry, particularly in the production of animal feed pellets. When compared with dry feed mixes, feed pellets have properties that are favored by the industry, such as improved feed quality, decreased pathogens, lower dust levels during manufacture, good handling, and more uniform ingredient dosing. Preferred industry pelleting processes utilize steam injection, in a process known as conditioning, which adds moisture and elevates the temperature prior to the pelleting step, which then forces the steam heated feed ingredients, or conditioned mash, through a die. The pelleting process temperatures may be from about 70°C to 95° C, or higher.
Enzymes are important feed ingredients and must be able to withstand increasingly higher processing temperatures used in pelleting processes, particularly those processes that employ expanders, while continuing to deliver *in vivo* efficacy.
Because of the steam, temperatures and compression forces used in pelleting processes, the stability of enzymes and other active agents is a problem that is illustrated by the fact that feed enzymes are often provided to the industry as stabilized liquid products that are added to feed pellets after the pelleting process to avoid enzyme inactivation. Homogeneous dosing is a problem when the enzyme is applied post pelleting, for instance, by spraying the enzyme onto the pellets, and the cost of the equipment to add enzyme post-pelleting is high. Alternatively, liquid enzyme formulations, or dry mix enzyme formulations are added to the mixer prior to pelleting. In certain instances, higher levels of enzymes than needed may be added in order to compensate for losses during pelleting.
Tablet forming processes also utilize compression forces and may or may not employ heat. Tablets are used in the household care industries, for instance, in laundry, dish and surface cleaning applications.
There is a need in the food, feed, and household care industries for stable, durable granules with active ingredients to serve as components in formulations that are subjected to steam treatment, for instance, pelleting and tableting processes, without appreciable loss of active agent activity and with dissolution profiles suitable to release the active ingredients to provide efficacy for their intended purpose. There also is a need for stable, durable granules with active agents that retain their activity when used as ingredients in animal feed formulations, such as unpelleted mixtures, that contain ingredients that adversely affect active agents.

SUMMARY OF THE INVENTION

The present invention relates to granules for feed compositions comprising: a core; an active agent; and at least one coating, the active agent of the granule retaining at least 50% activity, at least 60% activity, at least 70% activity, at least 80% activity after conditions selected from one or more of a) a feed pelleting process, b) a steam-heated feed pretreatment process, c) storage, d) storage as an ingredient in an unpelleted mixture, e) storage as an ingredient in a feed base mix or a feed premix, comprising at least one compound selected from trace minerals, organic acids, reducing sugars, vitamins, choline chloride, and compounds which result in an acidic or a basic feed base mix or feed premix.

In an embodiment of the present invention a granule for animal feed is provided comprising: a core; an active agent, the active agent of the granule retaining at least 80% activity after storage and after a steam-heated pelleting process where the granule is an ingredient; a moisture barrier coating; and a moisture hydrating coating that is at least 25% w/w of the granule, the granule having a water activity of less than 0.5 prior to the steam-heated pelleting process.

In yet another embodiment of the present invention, an animal feed ingredient is provided comprising: a granule comprising a core; an active agent surrounding the core; and at least one coating surrounding the active agent, the active agent retaining at least 50% activity, at least 60% activity, at least 70% activity, at least 80% activity after conditions selected from one or more of a) a feed pelleting process, b) a steam-heated feed pretreatment process, c) storage , d) storage as an ingredient in an unpelleted mixture, and e) storage as an ingredient in a feed base mix or a feed premix containing at least one compound selected from trace minerals, organic acids, reducing sugars, vitamins, choline chloride, and compounds which result in an acidic or a basic feed base mix or feed premix.

In a process of the present invention for producing a granule comprising an active agent for feed, the process comprises: preparing stable granules having a core, at least one active agent, and at least one coating; mixing the stable granules together with one or more of a) a diluent, b) a base mix, c) a premix, and d) a feed mixture for pelleting.

In another embodiment of the present invention, a process is provided for making a stable granule comprising enzyme for storage in a feed premix comprising choline chloride, the process comprising: providing a core material and enzyme, the enzyme distributed throughout the core material or layered over the core material; applying to the core material and enzyme a moisture hydrating material to form a layer that is at least 25% w/w of the stable granule; coating the layer with a moisture barrier material to form a coating that is at least 2% w/w of the granule, the applying and coating under conditions selected so that a water activity of the stable granule is less than 0.5.

Stable, durable granules of the above embodiments retain at least 50%, at least 60%, at least 70%, at least 80%, at least 90%, and at least 95% active agent activity under conditions of steam heated pelleting or pretreatment processes that raise the temperature of the pelleted material to as high as 85°C to about 95°C for up to about several minutes. The stable, durable granules of the present invention are particularly suitable as ingredients in animal feed pellets, and as ingredients in household care tablets.

In embodiments of the present invention, stable durable granules include a moisture hydrating material that constitutes at least about 55% w/w of the granule.

In other embodiments of the present invention, a stable durable granule has a moisture hydrating coating that comprising at least about 25%, about 30%, about 35%, about 40%, about 50%, about 55%, and about 60% w/w of the granule and a moisture barrier coating that comprises at least about 2% to about 40% w/w, about 2% to about 10%, about 2% to about 7%, and about 5% to about 15% of the granule.

In embodiments of the present invention, a stable durable granule includes three protective coatings wherein one coating comprises about 20% to 25% w/w of the granule of a moisture hydrating material and the two other coatings comprise about 5% to about 15% w/w of the granule of a moisture barrier material. In this embodiment, a heat annealing processing step is used to anneal the moisture barrier material.

In embodiments of the present invention, a stable durable granule comprises a silicate or clay core and an inherently thermal stable active agent within a matrix; an optional inorganic salt moisture hydrating protective coating; and an optional moisture barrier coating. In this embodiment, the optional coating layers constitute about 0% to about 15% w/w of the granule.

The present invention further includes methods for manufacturing the stable, durable granules as well as feed pellets, pet food, and household care and food tablets containing such stable, durable granules.

DETAILED DESCRIPTION OF THE INVENTION

The present invention provides stable, durable granules ,with active agents that withstand both high temperatures and compression forces when added to formulations subjected to, for instance, heated steam pretreatments, feed pelleting processes and tableting processes, while maintaining dissolution profiles suitable to release active agents that are able to provide efficacy for their intended purpose.

A first aspect of the present invention surprisingly provides stable durable granules with feed active agents that withstand steam pretreatments and feed pelleting steam-heated process temperatures and compression forces while maintaining dissolution profiles that release the feed active agents to provide intended efficacy. In this embodiment, granule components preferably are approved for use in feed.

A second aspect of the present invention provides stable, durable granules with enzymes that withstand steam-heated feed pretreatment and pelleting process temperatures and compression forces while maintaining dissolution profiles that release the enzyme to provide in *vivo* bioavailability efficacy. In this embodiment, granule components are edible by animals, and preferably are also digestible.

A third aspect of the present invention provides stable, durable granules with active agents for animal feed unpelleted mixtures, for example, premixes, The stable, durable granules retain efficacy when the unpelleted mixture is heat and steam pretreated prior to feeding to animals, or after the unpelleted mixture is pelleted. In this aspect of the invention, surprisingly, the active agents maintain activity when stored in unpelleted mixtures that contain ingredients that are detrimental to enzyme stability. In this aspect of the invention, without wishing to be bound by any particular theory, it is believed that a moisture hydrating material in the granules acts in combination with a moisture barrier material in the granules to protect the active ingredient from the detrimental ingredients in unpelleted mixtures. The moisture hydrating material retards or reduces the rate or extent of water migration into the area of the active agent, and the moisture barrier material excludes water. The combination of the moisture hydrating material and the moisture barrier material provides mechanical stability to further protect the active agent in the event that a layer of a moisture barrier material is damaged. Additionally, in some embodiments of the present invention, moisture barrier materials are used that oxidize only under extreme conditions, thereby in combination with moisture hydrating materials, the granules are chemically stable because it is believed that redox reactions are reduced during storage of the granules in unpelleted materials.

A fourth aspect of the present invention provides stable, durable granules with active agents that withstand tableting procedures while maintaining dissolution profiles that release the active agent to provide efficacy in household care applications, such as laundry, dish, and surface cleaning applications. In this embodiment, granule components may include materials that are not digestible by animals, for instance, surfactants, zeolites, bleaching materials, and colorants.

The stable, durable granules of the present invention are spherical or near spherical granules, although other shapes such as discs, ovals, cylindrical and oblong shapes may be used if desired. The granules have one or more protective layers surrounding the active agent.

The granules may be mixed together with dry ingredients, such as feed or household care formulations or unpelleted feed mixtures, for examples, pre-mix formulations, prior to use in a pelleting or tableting process, or used in dry feed mixtures and mash that are not pelleted. The granules are particularly suitable for use in feed pelleting manufacturing processes, and are also suitable for food, including pet food, and household care tablet manufacturing processes.

Unless defined otherwise, all technical and scientific terms used herein have the same meaning as commonly understood by one of ordinary skill in the art to which this invention pertains. As used in the specification and claims, the singular "a", "an" and "the" include the plural references unless the context clearly dictates otherwise. For example, the term granule may include a plurality of granules.

For purposes of this disclosure the "active agent" may be any material that is to be added to a stable, durable granule. The active agent may be a biologically viable material, a food or feed ingredient, an antimicrobial agent, an antibiotic replacement agent, a prebiotic, a probiotic, an agrochemical ingredient, such as a pesticide, fertilizer or herbicide; a pharmaceutical ingredient or a household care active ingredient, or combinations thereof. In a preferred embodiment, the active ingredient is a protein, enzyme, peptide, polypeptide, amino acid; carbohydrate, lipid or oil, vitamin, co-vitamin, or hormone, or combinations thereof. In another embodiment, the active ingredient is an enzyme, bleach, bleach activator, perfume, or other biologically active ingredient. Inherently thermostable active agents are encompassed by the present invention and exhibit enhanced thermostability in the granules of the present invention. Alternatively, less coating materials may be used when providing granules with inherently thermostable active agents, and the protective coating material amounts provided herein were selected primarily for active agents that were not inherently thermostable.

Most preferred active ingredients for food and feed applications are enzymes, peptides and polypeptides, amino acids, antimicrobials, gut health promoting agents, vitamins, and combinations thereof.

Any enzyme may be used, and a nonlimiting list of enzymes include phytases, xylanases, β-glucanases, phosphatases, proteases, amylases (alpha or beta or glucoamylases) cellulases, lipases, cutinases, oxidases, transferases, reductases, hemicellulases, mannanases, esterases, isomerases, pectinases, lactases, peroxidases, laccases, other redox enzymes and mixtures thereof.

Particularly preferred enzymes include a xylanase from *Trichoderma reesei* and a variant xylanase from *Tri-*

*choderma reesei,* both available from Danisco A/S, Denmark and/or Genencor International, Inc., Palo Alto, California, or the inherently thermostable xylanase described in EP1222256B1, as well as other xylanases from *Aspergillus niger, Aspergillus kawachii, Aspergillus tubigensis, Bacillus circulans, Bacillus pumilus, Bacillus subtilis, Neocallimastix patriciarum,Penicillium species, Streptomyces lividans, Streptomyces thermoviolaceus, Thermomonospora fusca, Trichoderma harzianum, Trichoderma reesei, Trichoderma viride*; examples of phytases are Finase L®, a phytase from Aspergillus sp., available from AB Enzymes, Darmstadt, Germany; Phyzyme™ XP, a phytase from E. Coli, available from Danisco, Copenhagen, Denmark, and other phytases from, for example, the following species: Trichoderma, Penicillium, Fusarium, Buttiauxella, Citrobacter, Enterobacter, Penicillium, Humicola, Bacillus, and Peniophora.

An example of a cellullase is Multifect® BGL, a cellulase (beta glucanase), available from Danisco A/S, Denmark, and other cellulases from species such as Aspergillus, Trichoderma, Penicillium, Humicola, Bacillus, Cellulomonas, Penicillium, Thermomonospore, Clostridium, and Hypocrea. The cellulases and endoglucanases described in US20060193897A1 also may be used. Amylases may be, for example, from species such as Aspergillus, Trichoderma, Penicillium, Bacillus, for instance, *B. subtilis, B. stearothermophilus, B. lentus, B. licheniformis, B. coagulans,* and *B. amyloliquefaciens.* Suitable fungal amylases are derived from *Aspergillus,* such as A. *oryzae* and *A. niger.* Proteases may be from *Bacillus amyloliquefaciens, Bacillus lentus, Bacillus subtilis, Bacillus licheniformis, and Aspergillus and Trichoderma species.*

Phytases, xylanases, phosphatases, proteases, amylases, esterases, redox enzymes, lipases, transferases, cellulases, and β-glucanases are enzymes frequently used for inclusion in animal feed. Enzymes suitable for inclusion into tablets for household care applications are similar, particularly proteases, amylases, lipases, hemicellulases, redox enzymes, peroxidases, transferases, and cellulases.

In particularly preferred aspects of the invention, the enzymes are selected from phytases, xylanases, beta glucanases, amylases, proteases, lipases, esterases, and mixtures thereof. In one embodiment of the present invention, two enzymes are provided in the granule, a xylanase and a beta-glucanase. The enzymes may be mixed together or applied to the granule separately. In another embodiment, three enzymes are provided in the granule, namely beta-glucanase, xylanase and phytase.

The above enzyme lists are examples only and are not meant to be exclusive. Any enzyme may be used in the durable granules of the present invention, including wild type, recombinant and variant enzymes of bacterial, fungal, yeast, plant, insect and animal sources, and acid, neutral or alkaline enzymes.

It will be recognized by those skilled in the art that the amount of enzyme used will depend, at least in part, upon the type and property of the selected enzyme and the intended use.

The durable granules of the invention include between about 0.0005 to about 20 % on a dry weight basis of the enzyme component of the granule. For instance, the weight percent of enzyme in embodiments of the invention comprises at least 0.0005 to about 15%, at least 0.001 to about 15%, at least 0.01 to about 10%, at least 0.1 to about 10%, at least 1.0 to about 10%, at least 1.0 to about 8%, at least 1.0 to about 5%, and at least 2.0 to at least 5% in the granule. Typical doses of 25to 400 grams of the stable, durable enzyme granules per ton of feed will deliver about 0.0001 to about 80 grams of active enzyme protein per ton of feed, and the enzyme granules may be dosed as high as 5000 grams per ton of feed. Dosages for other active ingredients typically are 0.001 to about 400 grams/ton of the feed, or higher. Dosages may be even higher when the thermostable granules of the present invention are used as a component of animal feed unpelleted mixtures, for example, pre-mixes, which may contain several active agents and are added to feed compositions. For example, the dosage of the enzyme granules added to a premix may be about 0.2% to 10% of the premix, or about 0.1% to about 3% of a base mix. In an exemplary embodiment, the activity level of phytase containing stable, durable granules stored in a premix is about 500 U/g, or higher. Premixes typically are added to the diet at about 0.5% to about 2%, and base mixes are added at about 2% to about 6%.

The portion of the durable granule, without the protective coating(s) of the invention, and comprising the active ingredient, including any processing solids, binders and other ingredients therein, may comprise less than about 70%, less than about 60%, less than about 50%, less than about 40%, less than about 30%, and less than about 20% by weight of the granule, with, generally, the weight percent being about 25% to about 50% w/w, about 40% to about 60%, or about 50% to about 60%.

The coating layer(s) may comprise more than about 30%, more than about 40%, more than about 50%, more than about 60%, more than about 70%, and more than about 80% by weight of the granule, with, generally, weight percent being about 50% to about 75% w/w, 40% to about 60%, or about 40% to about 50%, depending upon the type and number of coating layers.

For embodiments that utilize an inherently thermostable active agent, the core may constitute as much as about 85 to about 100% w/w of the granule and the coating may constitute about 0% to about 15% w/w of the granule.

The durable granules of the present invention may be sized as desired and are between about 300 um to about 1000 um in diameter, about 300 um to about 900 um, about 400 um to about 800 um, and about 400 um to about 600 um.

"Compression forces." for purposes of this disclosure refers to generally axially applied forces that cause the atoms of a material to compact. Compression forces, as used herein, occur in pelleting and tableting processes and may include an element of flexural stress where the forces applied are not completely symmetrical with respect to the longitudinal axis of the material.

An "inherently thermostable enzyme or protein" refers to enzymes and proteins having a melting point that is above about 60° C to about 65° C, for example, the inherently thermostable enzyme utilized in Table 2 for granules 28, 29 and 30, has a melting point of 69° C at a pH of 5.5 and a melting point of 72° C at a pH of 8.0. A number of the thermolabile enzymes referred to throughout the specification had, for example, a melting point of less than 60° C at pH values of 5.5 and 8.0.

"Moisture barrier materials" refers to materials that exclude, prevent or substantially retard water uptake. These materials typically are hydrophobic or amphiphilic, provide insulation against water and do not inherently absorb and/or bind water and include, but are not limited to, film-forming materials. Examples of moisture barrier materials include polymers, proteins, lipids, fats and oils, fatty acids and gums. Examples of film forming moisture barrier materials are natural and modified polymers, such as gum arabic, whey, whey protein concentrate, PVA, including modified PVA and synthetic polymers such as , latex, HPMC, and acid-thinned hydroxypropyl starch, for example; PureCote™, oxidized starch, and modified starch. Non-film forming moisture barrier materials include, for instance, waxes, fats, oils and lipids, and lecithin. Selected moisture barrier materials that do not readily oxidize are, for example, latex polymer and polymers such as gum arabic.

"Moisture hydrating materials" refers to materials that take up aqueous liquids, such as water, by one several mechanisms. In a first mechanism, the materials absorb free water. In a second mechanism, the materials take up bound water that generally is present as crystalline waters of hydration. Accordingly, the materials may be provided as partially or fully hydrated materials or as non-hydrated materials that will absorb or bind aqueous liquids and retard or reduce the rate or extent of migration of such liquids to the active agent. In a third mechanism, moisture hydrating materials thermally insulate the active agent by retarding heat transfer to the active agent within the granule and by maintaining the active agent at a lower temperature than the temperature at the exterior surface of the granule. Moisture hydrating materials include carbohydrates and inorganic salts, including hydrated salts, such as magnesium sulfate, sodium sulfate, and ammonium sulfate; maltodextrin; sugars, for example, sucrose; and cornstarch.

In exemplary embodiments of the present invention, the moisture hydrating materials are inorganic salts which, when added to the granule at greater than about 25% w/w, are anhydrous or contain relatively low amounts of bound or free water so that the water activity of the completed granule is less than 0.5. Without wishing to be bound by any particular theory, when the granule is subjected to steam-heating processes, the inorganic salt moisture hydrating material will begin to take up water from the steam-heated treatment, the water moving into the moisture hydrating material in a kinetic process over the short time period of the steam-treatment to prevent the water from penetrating into the area of the granule bearing the active agent. In these embodiments with a water activity of the granule of less than 0.5, the moisture hydrating material may constitute as little as 25% w/w of the granule. In embodiments with moisture hydrating material constituting about 40-70% w/w of the granule, the thicker layer of the moisture hydrating material allows for the use of hydrated or partially hydrated materials, particularly inorganic salts. For purposes of this disclosure, "hydrated", "partially hydrated", and "non-hydrated" refer to a material's hydration potential when the granule is at equilibrium prior to being subjected to steam-heating. A "hydrated" material refers to a material that contains water in a free or bound form, or a combination thereof. Water may be added either during or after coating processes and the degree of hydration of the granule is a function of the granule materials and the temperature, humidity and drying conditions under which it is applied.

"Pellets" and "Pelleting" refer to solid rounded, spherical and cylindrical tablets or pellets and the processes for forming such solid shapes, particularly feed pellets and solid, extruded animal feed. Known feed pelleting manufacturing processes generally include mixing together feed ingredients for about 1 to about 5 minutes, transferring the mixture to a surge bin, conveying the mixture to a steam conditioner, optionally transferring the steam conditioned mixture to an expander, transferring the mixture to the pellet mill or extruder, and finally transferring the pellets into a pellet cooler. Fairfield, D. 1994. Chapter 10, Pelleting Cost Center. In Feed Manufacturing Technology IV. (McEllhiney, editor), American Feed Industry Association, Arlington, VA, pp. 110-139.

The steam conditioner treats the mixture for about 20 to about 90 seconds, and up to several minutes, at about 85°C to about 95°C. The amount of steam may vary in accordance with the amount of moisture and the initial temperature of the feed mix. About 4% to about 6% added steam has been reported in pelleting processes, and the amount is selected to produce less than about 18% moisture in the mash prior to pelleting, or up to about 28% moisture in mash intended for extrusion.

An optional expander process occurs for about 4 to about 10 seconds at a temperature range of about 100°C to about 140°C. The pellet mill portion of the manufacturing process typically operates for about 3 to about 5 seconds at a temperature of about 85°C to about 95°C.

A "stable" granule refers to a granule in which the activity of the active agent(s) is substantially maintained after inclusion as an ingredient in a formulation subjected to steam heated pretreatment processes, steam heated pelleting processes, steam heated tableting processes, after storage alone and storage as an ingredient in unpelleted and untableted mixtures. Stability includes thermostability, shelf-life or storage stability, mechanical stability, and/or chemical stability when the active agent containing granules are stored in unpelleted or untableted mixtures and/or subjected to steam-heated and pressurized pelleting processes and tabletting processes. Mechanical stability refers to the physical robustness or structural integrity of granules, which structural integrity includes resistance to microbials, resistance to dust production, and resistance to release of ingredients that may result in the production of odors. Chemical stability refers to the substantial maintenance of activity when the granules are stored in unpelleted or untableted mixtures with ingredient(s) harmful to the active agent(s). Thermostability is defined further herein, and generally refers to maintenance of activity following exposure to temperatures up to about 85° C to 95° C when the stable, durable granules are an ingredient of pellets, tablets and unpelleted and untableted mixtures.

"Tableting" refers to processes for forming solid slugs or tablets by compressing a mixture of ingredients in a tablet press as described in EP 1 257 631B1, which is hereby incorporated by reference.

Tablets may be made by direct compression tableting of mixtures of active agents, fillers/binders, lubricants, and any other optional ingredients. The active agent component is mixed thoroughly with the other tablet ingredients prior to entering the tablet machine. Ingredients are blended in any suitable mixing device, such as a twin shell blender or similar apparatus, or using any mixing method that results in blending of the tablet ingredients.

The mixtures are then compressed into tablets, using any tableting device, such as a tablet press (Stokes Model R-4, Warminster, PA). Tablet presses generally have upper and lower shape-corresponding punches, which fit into a die from above and below the die. Mixed tablet material is filled into the die cavity and at least one of the punches, typically the upper punch, enters the die cavity. Pressure is applied to both the upper and lower punches. The action of the upper and lower punches moving toward each other, applies pressure to the material between the punches, thus forming a tablet.

A wide variety of tablet shapes can be made. Tablet shape is determined by the tooling of the punches. Compaction forces vary, depending on the punch geometry, type of instrument, and formulation used. Alternatively, tablets may be made using dry or wet granulation procedures as described in US Pat. No. 6,852,336, which is hereby incorporated by reference herein in its entirety. The '336 patent states that dry granulation procedures may be utilized where one of the components has sufficient cohesive properties to be tableted. The method mixes the ingredients with a lubricant, if required. The wet granulation procedure described mixes the dry ingredient using a twin shell blender or double-cone blender under shear mixing conditions and then adds solutions of a binding agent to the mixed powders to obtain a granulation.

A "thermostable" granule refers to a granule having an active agent that retains at least 50%, at least 60%, at least 70%, at least 80%, at least 90%, and at least 95% of its activity, measured by a conventional assay specific for the selected active agent, following inclusion in a formulation, and exposure to heat or to heat and steam, such that the formulation reaches temperatures up to about 85°C to about 95°C for up to several minutes. Those skilled in the art will recognize that thermostable granules in formulations exposed to temperatures less than 85° C are stable, and test results conducted at 80° C establish that this is indeed the case. Accordingly, it will be understood that up to 85° C to 95° C is meant to encompass temperatures less than 85° C, which are included in the scope of this invention.

In one embodiment, "thermostable" refers to an active agent that retains at least 80% of its activity measured by a conventional assay specific for the selected active ingredient following inclusion in a formulation, exposed to steam, such that the formulation reaches temperatures of about 85°C-95°C for about 30 seconds. In yet another embodiment, thermostable refers to an active agent that retains at least 50% of its activity measured by a conventional method that is specific for the selected active ingredient following inclusion in a formulation, exposed to steam, such that the formulation reaches temperatures of about 85° C-95°C for about 30 seconds.

"Unpelleted mixtures" refers to premixes or precursors, base mixes, mash, and diluents. Premixes typically contain vitamins and trace minerals. Base mixes typically contain food and feed ingredients such as dicalcium phosphate, limestone, salt and a vitamin and mineral premix, but not grains and protein ingredients. Diluents include, but are not limited to grains (for example wheat middlings and rice bran) and clays, such as phyllosilicates (the magnesium silicate sepiolite, bentonite, kaolin, montmorillonite, hectorite, saponite, beidellite, attapulgite, and stevensite). Clays also function as carriers and fluidizing agent, or diluents, for feed premixes. Mash typically comprises a complete animal diet.

The stable granules of the present invention may be added to these unpelleted mixtures, and to mash mixtures, which may be subsequently treated with steam and/or steam pelleted or dried.

"Water activity", symbolized as $a_w$ refers to the fractional relative humidity of an atmosphere in equilibrium with a solid or liquid phase material, i.e., the ratio of the partial pressure of water vapor to that present above pure water at the same temperature. In all phases between which water distribution has reached equilibrium, it is by definition equal. The term "relative humidity" is generally used to describe the water in the atmosphere or gas phase in equilibrium with the solid, and is expressed as a percentage, with 100% as the relative humidity of pure water in a closed system. Thus, for any water activity value, there is a corresponding relative humidity given by $\%RH=100*a_w$. Water activity can be readily measured by methods known in the art, typically by placing a sample of the material inside the temperature-controlled chamber of a water activity meter, such as the water Activity System Model D2100 available from Rotronic Instrument Corp. (Huntington, N.Y.), and allowing the measurement to reach equilibrium as indicated on the display. The water activity referred to herein is that of the granule itself after application of all coatings.

Preferred water activity for the granules of the present invention is, particularly when polymer outer coatings are to be used, less than 0.5.

The stable, durable granule of the present invention is a granule that may be prepared by any process that results in the application of one or more protective coatings to a core. The active agent may be a component of the core of the granule, or may be coated over a core material, and for the purposes of this disclosure, a core refers to all components of the durable granule except any protective coating(s) applied to the core of the granule.

The core may be made by any process known in the art, such as granulation, extrusion, pan coating, spheronization, drum granulation, high-shear agglomeration, fluid-bed spray coating, crystallization, precipitation, and prill processes. Such processes are known in the art and are described in US Pat. No. 4,689,297 and US Pat. No. 5,324,649 (fluid bed processing); EP656058B 1 and US Pat. No. 454332 (extrusion process); US Pat. No. 6,248,706 (granulation, high-shear); and EP804532B1 and US Pat. No. 6,534466 (combination processes utilizing a fluid bed core and mixer coating), all disclosures of which are incorporated by reference in their entirety.

The protective coatings of the present invention may be applied as described in the granulation, extrusion, fluid-bed and prill processes noted above. Additionally, the protective coatings may be applied by casting methods, including spinning disk casting methods, as described in WO 03/000625, which is hereby incorporated by reference in its entirety.

Additionally, in one embodiment, the process may include a heat annealing step, which heats the granule to above the glass transition temperature (Tg) of a moisture barrier component and then gradually reduces the temperature to cause the barrier material to harden, or become glassy.

CORES

The core is the inner nucleus of the granule, and as stated above, may include the active ingredient or the active ingredient may be coated around a core material. Suitable cores for use in the present invention are preferably a hydratable or porous material (i.e., a material which is dispersible or soluble in water) that is a feed grade material. The core material should either disperse in water (disintegrate when hydrated) or solubilize in water by going into a true aqueous solution. Clays (for example, the phyllosilicates bentonite, kaolin, montmorillonite, hectorite, saponite, beidellite, attapulgite, and stevensite, silicates, such as sand (sodium silicate), nonpareils and agglomerated potato starch or flour, or other starch granule sources such as wheat and corm cobs are considered dispersible. Nonpareils are spherical particles consisting of a seed crystal that has been built onto and rounded into a spherical shape by binding layers of powder and solute to the seed crystal in a rotating spherical container. Nonpareils are typically made from a combination of a sugar such as sucrose, and a powder such as cornstarch. In one embodiment of the present invention the core is a sodium chloride or sodium sulfate crystal, sometimes referred to as a seed, or other inorganic salt crystal. In another embodiment of the present invention, the core is a sucrose crystal.

Particles composed of inorganic salts and/or sugars and/or small organic molecules may be used as the cores of the present invention. Suitable water soluble ingredients for incorporation into cores include: inorganic salts such as sodium chloride, ammonium sulfate, sodium sulfate, magnesium sulfate, zinc sulfate; or urea, citric acid, sugars such as sucrose, lactose and the like.

Cores of the present invention may further comprise one or more of the following: active agents, feed or food grade polymers, fillers, plasticizers, fibrous materials, extenders and other compounds known to be used in cores. Suitable polymers include polyvinyl alcohol (PVA), polyethylene glycol, polyethylene oxide, polyvinyl pyrrolidine, and carbohydrate polymers (such as starch, amylose, amylopectin, alpha and beta-glucans, pectin, glycogen), including mixtures and derivatives thereof.

Cores used in the examples include sugar crystals, inorganic salt crystals, corn cob cores, clays and silicate.

Suitable fillers useful in the cores include inert materials used to add bulk and reduce cost, or used for the purpose of adjusting the intended enzyme activity in the finished granule. Examples of such fillers include, but are not limited to, water soluble agents such as salts, sugars and water dispersible agents such as clays, talc, silicates, cellulose and starches, and cellulose and starch derivatives.

Suitable plasticizers useful in the cores of the present invention are low molecular weight organic compounds and are highly specific to the polymer being plasticized. Examples include, but are not limited to, sugars (such as, glucose, fructose and sucrose), sugar alcohols (such as, sorbitol, xylitol and maltitol and other glycols), polar low molecular weight organic compounds, such as urea, or other known plasticizers such as water or feed grade plasticizers.

Suitable fibrous materials useful in the cores of the present invention include, but are not limited to: cellulose, and cellulose derivatives such as HPMC (hydroxypropyl-methyl cellulose), CMC (carboxy-methyl cellulose), HEC (hydroxy-ethyl cellulose).

In one embodiment, particularly for feed applications, of the present invention, the core is a water-soluble or dispersible corn cob material or sugar or salt crystal. In another embodiment particularly suitable for household cleaning applications, the core is a water-soluble or dispersible sugar or salt crystal or a non pareil.

Those skilled in the art will recognize that, for feed and food applications, the cores (and any polymers, fillers, plasticizers, fibrous materials, and extenders), are acceptable for food and/or feed applications. For household cleaning applications, such a restriction need not apply.

The core of the granules of the present invention, including any coating having active ingredients therein, and excluding the protective coatings described below, preferably comprises less than about 70%, less than about 60%, less than about 50%, less than about 40%, less than about 30%, and less than about 20% w/w of the durable granule.

ACTIVE AGENT

The active agent or agents, particularly the enzymes discussed above, is obtained from a fermentation broth and may be a whole broth, lysed broth, or clarified fermentation broth recovered from the fermentation process. The enzyme may be in liquid, slurry, dried, lyophilized, or crystalline form as obtained using recovery processes from fermentation broths. The enzyme(s) optionally are mixed together with plasticizers, such as carbohydrates, and polymers, such as modified, or native starch to form a matrix. A non-limiting list of plasticizers includes the sugars glucose, fructose and sucrose, dextrins, PVA, and sugar alcohols, such as, sorbitol, xylitol and maltitol, or any of the plasticizers listed above. Modified starches include, but are not limited to cornstarch and acid-thinned hydroxy propyl starch, for example, PureCote®, and oxidized starches. Alternatively, the active agent may be added to the core, or added to both the core and a layer surrounding the core.

PROTECTIVE COATINGS

The protective coatings of the present invention generally are applied as one or more layers surrounding the core when the active agent is not inherently thermostable. Embodiments include one, two, three or four protective coating layers.

Suitable protective coating materials are polymers, carbohydrates, proteins, lipids, fats and oils, fatty acids, inorganic salts, and gums and mixtures thereof.

The protective costings include moisture barrier coatings and moisture hydrating coatings. The moisture barrier coatings function by excluding moisture, for instance by forming a shell layer that typically does not absorb moisture and prevents or retards the rate of moisture migration into the granule. Moisture hydrating coatings on the granule absorb or bind moisture as either free water or water of hydration, thereby acting to impede or retard the extent or rate of transport of external moisture into the granule. The moisture hydrating coatings typically constitute at least about 35% w/w of the granule. The moisture hydrating materials in the coatings thermally insulate the active agents and will absorb a certain amount of moisture and retain it within the hydrating material without allowing it to pass through into the portion of the granule having the active agent. For moisture hydrating coatings on stable, durable granules that do not contain appreciable amounts of water prior to steam treatment, such coatings may constitute about 25% w/w of the granule.

Moisture barrier coatings typically comprise hydrophobic materials, such as hydrophobic polymers, for example PVA, HPMC, acid-thinned hydroxypropyl starches and oxidized starch; proteins, for example whey and whey protein concentrates; lipids, for example, lecithin; fats and oils, fatty acids, latex and gums, for example, gum arabic. Certain moisture barrier coatings, such as PVA and gum arabic, are not readily oxidized and find particularly applicability in providing chemical stability when the granules of the invention are stored in unpelleted or untableted mixtures, for instance, in premixes that contain choline chloride. Moisture hydrating coating materials typically are hydrophilic materials, such as carbohydrates and inorganic salts, including hydrated salts. Examples of moisture hydrating materials are magnesium sulfate, sodium sulfate, maltodextrin, ammonium sulfate, sugars, for example, sucrose, and native Cornstarch.

Polymers used for the protective coatings are polyvinyl alcohol (PVA), polyethylene glycol, polyvinyl pyrrolidone, polyacrylates, polyethylene oxides (PEO), polylactic acid, polyvinylchloride, polyvinylacetate, polyvinyl pyrrolidones (PVP), cellulose others, alginates, gelatin, modified starches and substituted derivatives, hydrolysates and copolymers thereof such as acid-thinned hydroxypropyl starch, such as, Pure Cote™, hydroxypropyl methyl cellulose (HPMC), methyl cellulose (MC), carboxymethyl cellulose (CMC), and ethyl cellulose. Most Preferred polymers for the protective coatings are PVA, modified PVA, as described in U,S. Pat. NO. 6,872,696, and modified cellulose, such as methyl cellulose and hydroxylpropylmethyl cellulose, as described in PCT Publication No. WO 99/51210, both of which are incorporated by reference herein

Carbohydrates used for the protective coatings are maltodextrin hydroxylmethyl cellulose, modified or native starches made from corn, sorghum, arrowroot, rice, wheat, rye, barley, oat, potato, yam, tapioca, cassava, sago, and sugars including sucrose, corn syrup solids, molasses, glucose, fructose, and lactose.

Proteins used for the protective coatings are whey powder, whey protein concentrate, whey protein isolate, caseinates, soy protein concentrate and isolate, zein, albumin and gelatin.

Simple, compound and derived lipids that may be used in the protective coatings are waxes (for example, vegetable, mineral and synthetic, such as carnauba, candelilla, beeswax, cerumen, carnuba, shellac, paraffin, and microcrystalline waxes); lecithin (for example mono-and diglycendes); fatty acids (for example stearic, palmitic, linoleic, oleic, butyric, and arachidonic fatty acids and their salts of sodium, potassium, calcium and zinc); and fats and oils (for example, hydrogenated or partially hydrogenated fats and oils, such as soy, corn, cottonseed, tallow, canola, and linseed oil). A preferred lipid for the protective coatings is lecithin.

Inorganic salts used for the protective coatings include salts of sulfate, citrate, chloride, carbonate, sulfite, phosphate, phosphonate, and bicarbonate salts of sodium, ammonium, potassium, calcium, magnesium and zinc. Preferred salts are magnesium, sodium and ammonium sulfates.

Gums that may be used in the protective coatings include gum arable, guar gum, agar, gum tragacanth,karya gum, locust bean gum, carageenan, xanthan gum, and alginates.

The protective coatings of the present invention further may include plasticizers, lubricants, pigments and powders, such as talc, bentonite, kaolin, cornstarch, magnesium silicate, calcium carbonate, and chitosan.

Certain embodiments of the present invention typically have a single layer of a moisture hydrating material that is approximately at least 55% w/w of the granule. Because the capacity of moisture hydrating coatings to take up and sequester water has a limit, relatively high levels of single layer coatings are applied. Alternatively, moisture hydrating material(s) may be applied in two layers. Other embodiments of the present invention have protective coatings utilizing both moisture hydrating materials and moisture barrier materials. In these embodiments, the amount of moisture hydrating material may be lower, at least about 25% w/w of the granule and the moisture barrier material is about 2% to 25% w/w of the granule. Using both moisture hydrating materials and moisture barrier materials combines protective mechanisms and typically reduces cost, particularly of the moisture barrier materials. Moisture barrier materials, particularly film-forming materials may be subject to mechanical damage which, if these materials are used alone as a thin coating, may lead to loss of protection for the active agent. The combination allows for the use of less of both materials than would be required if the materials were used alone. The combination allows for some damage to the moisture barrier layer in view of the presence of the moisture hydrating material. As discussed above, the combination of the materials is particularly suitable to provide chemical stability by maintaining activity when the granules are stored in unpelleted mixtures.

For granules having a heat annealing processing step, the amount of moisture hydrating material may be lowered to about at least 20% w/w of the granule Because the fused moisture barrier layer has improved continuity and is less subject to mechanical damage.

Processes of the present invention, in addition to the processes described for manufacture of the stable, durable granules, include mixing of the granules with unpelleted feed mixtures and storage of the resulting mixtures. Additional processes include steam-treating the resulting mixtures and/or pelleting the resulting mixtures. Of course the stable, durable granules of the present invention also may be stored alone and mixed with feed, or pelleted, when desired.

The following examples are not meant to be limiting.

EXAMPLES

Table 1 below is a list of ingredients and abbreviations used in the following examples.

Table 1

| Ingredient | Name & Product No. | Supplier |
|---|---|---|
| Sucrose | Domino Pure Cane Extra Fine Granulated Sugar | Tate & Lyle North American Sugars, Baltimore, MD |
| Cornstarch | Corn Starch | Cargill Foods, Minneapolis, MN |
| MgSO4 | Magnesium sulfate heptahydrate, MgSO4 7H2O (Epsom salts) | PQ Corporation, Berwyn, PA |
| HPMC | Hydroxypropyl Methylcellulose, Brandname: METHOCEL | Dow Chemical |
| Carnauba wax | Michem Lube 160HS (camauba emulsion, 50% solids) | Michelman Inc. Cincinnati, OH |
| Modified starch | PURE-COTE® | Grain Processing Corporation, Muscatine, IA |
| Lecithin | ULTRALEC® P Soy lecithin | ADM Corp., Decatur, IL |

(continued)

| Ingredient | Name & Product No. | Supplier |
|---|---|---|
| PVA | polyvinyl alcohol, Elvanol® 51-05 | DuPont, Wilmington, DE |
| Sodium sulfate | Sodium Sulfate Anhydrous | Cooper Natural, Tulsa, Oklahoma |
| Ammonium sulfate | Ammonium Sulfate CAS # 7783-20-2 | General Alum & Chemical Corp. Searaport, Maine |
| Talc | NYTAL 4000 TALC | RT Vanderbilt Co, Inc. Norwalk, CT |
| Whey powder | PC42010 | Leprino Foods, Denver CO |
| Whey protein | Proliant instantized whey protein concentrate, #8010, lot H4212 | Hilmar Cheese Company, Hilmar, CA (WPC formally sold through Proliant) |
| Linseed oil | ASTM Raw Linseed Oil | Cargill Industrial Oils, Chicago, IL |
| Maltodextrin | Maltodextrin M150 | Grain Processing Corp (GPC) Muscatine, IA |
| Gum Arabic | TIC PRETESTED Gun Arabic FT PreHydrated | TIC Gums, Belcamp, MD |
| Canola oil | Canola Oil | Safeway |
| Latex | Aquacoat ECD (Ethylcellulose dispersion 30%) | FMC BioPolymer. Philadelphia, PA |
| Sand | Sodium silicate | Sigma-Aldrich Chemical Co. |
| Corn cobs | Ground corn cobs or corn pith | ICBP independence, IA |

Table 2 illustrates the composition of a number of exemplary stable, durable granules of the present invention and several granules that did not demonstrate at least 50% recovered active agent activity under particular pelleting conditions.

Table 2

| Formulation #1* | | |
|---|---|---|
| | Component | % (w/w) dry basis |
| Core Material Active Agent | sucrose 35-50 mesh | 36.9 |
| | Enzyme | 7.4 |
| | sucrose | 12.6 |
| | Cornstarch | 12.6 |
| 1st coating | Sucrose | 10.5 |
| | Cornstarch | 10.5 |
| 2nd Coating | carnuaba wax | 6.0 |
| 3rd Coating | HPMC | 3.5 |
| *Heat annealing step | | |
| Formulation #2 | | |
| | Component | % (w/w) dry basis |
| Core Material Active Agent | sucrose 35-50 mesh | 17.5 |
| | enzyme | 3.5 |
| | sucrose | 6.0 |
| | cornstarch | 6.0 |
| 1st Coating | MgSO4 | 67.0 |

(continued)

| Formulation #3 | | |
|---|---|---|
| | Component | % (w/w) dry basis |
| Core Material Active Agent | sodium sulfate | 16.5 |
| | enzyme | 4.8 |
| | sucrose | 4.0 |
| | cornstarch | 8.0 |
| 1st Coating | MgSO4 | 66.7 |

| Formulation #4 | | |
|---|---|---|
| | Component | % (w/w) dry basis |
| Core Material Active Agent | sucrose 35-50 mesh | 17.5 |
| | enzyme | 3.5 |
| | sucrose | 4.0 |
| | cornstarch | 8.0 |
| 1st Coating | maltodextrin | 67 |

| Formulation #5 | | |
|---|---|---|
| | Component | % (w/w) dry basis |
| Core Material Active Agent | sodium sulfate | 14.8 |
| | enzyme | 2.9 |
| | sucrose | 3.3 |
| | cornstarch | 6.6 |
| 1st Coating | MgSO4 | 55.6 |
| 2nd Coating | arabic | 17.0 |

| Formulation #6 | | |
|---|---|---|
| | Component | % (w/w) dry basis |
| Core Material Active Agent | sodium sulfate | 14.8 |
| | enzyme | 2.9 |
| | sucrose | 3.3 |
| | cornstarch | 6.6 |
| 1st Coating | MgSO4 | 55.6 |
| 2nd Coating | whey | 16.7 |

| Formulation #7 | | |
|---|---|---|
| | Component | % (w/w) dry basis |
| Core Material Active Agent | sodium sulfate | 14.8 |
| | enzyme | 2.9 |
| | sucrose | 3.3 |
| | cornstarch | 6.6 |
| 1st Coating | MgSO4 | 55.6 |
| 2nd Coating | whey protein concentrate | 17.0 |

| Formulation #8 | | |
|---|---|---|
| | Component | % (w/w) dry basis |
| Core Material Active Agent | sodium sulfate | 13.4 |
| | enzyme | 2.6 |
| | sucrose | 3.0 |
| | cornstarch | 6.0 |
| 1st Coating | PureCote® starch | 15.0 |

| | lecithin | 10.0 |
|---|---|---|
| 2nd Coating | lecithin | 10.0 |
| 3rd Coating | MgSO4 | 50.0 |

| Formulation #9 | | |
|---|---|---|
| | Component | % (w/w) dry basis |
| Core Material Active Agent | sodium sulfate | 9.1 |
| | enzyme | 1.8 |
| | sucrose | 2.0 |
| | cornstarch | 4.1 |
| 1st Coating | MgSO4 | 83.0 |

| Formulation #10 (Thermolabile) | | |
|---|---|---|
| | Component | % (w/w) dry basis |
| Core Material Active Agent | sucrose 35-50 mesh | 26.5 |
| | enzyme | 5.3 |
| | sucrose | 9.1 |
| | cornstarch | 9.1 |
| 1st Coating | carnuaba wax | 50 |

| Formulation #11 | | |
|---|---|---|
| | Component | % (w/w) dry basis |
| Core Material Active Agent | sodium sulfate | 10.7 |
| | enzyme | 2.1 |
| | sucrose | 2.4 |
| | cornstarch | 4.8 |
| 1st Coating | PVA | 4 |
| | talc | 36 |
| 2nd Coating | MgSO4 | 40 |

| Formulation #12 (Test granule with no protective coating) | | |
|---|---|---|
| | Component | % (w/w) dry basis |
| Core Material Active Agent | sucrose 35-50 mesh | 53.1 |
| | enzyme | 10.6 |
| | sucrose | 18.1 |
| | cornstarch | 18.1 |

| Formulation #13 (Thermolabile) | | |
|---|---|---|
| | Component | % (w/w) dry basis |
| Core Material Active Agent | sucrose 35-50 mesh | 44.2 |
| | enzyme | 8.9 |
| | sucrose | 15.1 |
| | cornstarch | 15.1 |
| 1st Coating | sucrose & starch (1:1 ratio) | 16.7 |

| Formulation #14 (Thermolabile) | | |
|---|---|---|
| | Component | % (w/w) dry basis |
| Core Material Active Agent 1st coating | sucrose 35-50 mesh | 48.3 |
| | enzyme | 9.7 |
| | sucrose | 16.5 |
| | cornstarch | 16.5 |
| 1st Coating | carnuaba wax | 9.1 |

| Formulation #15 (Thermolabile) | | |
|---|---|---|

|  | % Component | (w/w) dry basis |
|---|---|---|
| Core Material Active Agent | sucrose 35-50 mesh | 49.6 |
|  | enzyme | 9.9 |
|  | sucrose | 16.9 |
|  | cornstarch | 16.9 |
| 1st Coating | ethylcellulose | 11.5 |

| Formulation #16 (Thermolabile) | | |
|---|---|---|
|  | Component | % (w/w) dry basis |
| Core Material Active Agent | sucrose 35-50 mesh | 48.3 |
|  | enzyme | 9.7 |
|  | sucrose | 16.5 |
|  | cornstarch | 16.5 |
| 1st Coating | hydroxypropylmethylcellulose | 9.1 |

| Formulation #17 | | |
|---|---|---|
|  | Component | % (w/w) dry basis |
| Core Material Active Agent | sodium sulfate | 10.7 |
|  | enzyme | 2.1 |
|  | sucrose | 2.4 |
|  | cornstarch | 4.8 |
| 1st Coating MgSO4 |  | 40 |
| 2nd Coating PVA |  | 4 |
|  | talc | 36 |

| Formulation #18 | | |
|---|---|---|
|  | Component | % (w/w) dry basis |
| Core Material Active Agent | sodium sulfate | 13.4 |
|  | enzyme | 2.6 |
|  | sucrose | 3.0 |
|  | cornstarch | 6.0 |
| 1st Coating | MgSO4 | 50.0 |
| 2nd Coating lecithin |  | 10.0 |
|  | 3rd Coating PureCote® starch | 15.0 |

| Formulation #19 | | |
|---|---|---|
|  | Component | % (w/w) dry basis |
| Core Material Active Agent | sodium sulfate | 16.6 |
|  | enzyme | 4.8 |
|  | sucrose | 4.0 |
|  | cornstarch | 0.0 |
|  | talc | 8.0 |
| 1st Coating | MgSO4 | 66.7 |

| Formulation #20 | | |
|---|---|---|
|  | Component | % (w/w) dry basis |
| Core Material Active Agent | sodium sulfate | 14.8 |
|  | enzyme | 2.9 |
|  | sucrose | 3.3 |
|  | cornstarch | 6.6 |
| 1st Coating | gum arabic | 17.0 |
| 2nd Coating | MgSO4 | 55.6 |

| Formulation #21 (Thermolabile) | | |
| --- | --- | --- |
| | Component | % (w/w) dry basis |
| Core Material Active Agent | sucrose 35-50 mesh | 51.9 |
| | enzyme | 10.4 |
| | sucrose | 17.7 |
| | Cornstarch | 17.7 |
| 1st Coating | canola oil | 2.2 |

| Formulation #22 | | |
| --- | --- | --- |
| | Component | % (w/w) dry basis |
| Core Material Active Agent | ground corncob | 17.5 |
| | enzyme | 3.5 |
| | sucrose | 6.0 |
| | cornstarch | 6.0 |
| 1st Coating | MgSO4 | 67 |

| Formulation #23 | | |
| --- | --- | --- |
| | Component | % (w/w) dry basis |
| Core Material Active agent | sodium sulfate | 31.1 |
| | enzyme | 2.4 |
| | cornstarch | 9.4 |
| 1st Coating | MgSO4 | 43.7 |
| 2nd Coating | Gum Arabic | 3.4 |
| | maltodextrin | 10.1 |

| Formulation #24 (Thermolabile) | | |
| --- | --- | --- |
| | Component | % (w/w) dry basis |
| Core Material Active agent | sodium sulfate | 29.9 |
| | enzyme | 3.3 |
| | cornstarch | 9.3 |
| 1st Coating | PureCote | 12.0 |
| 2nd Coating | Lecithin | 8.0 |
| 3rd Coating | MgSO4 | 37.5 |

| Formulation #25 (Thermolabile) | | |
| --- | --- | --- |
| | Component | % (w/w) dry basis |
| Core Material Active agent | sodium sulfate | 47.8 |
| | Enzyme | 5.3 |
| | cornstarch | 14.9 |
| 1st Coating | PureCote | 19.2 |
| 2nd Coating | Lecithin | 12.8 |

| Formulation #26 | | |
| --- | --- | --- |
| | Component | % (w/w) dry basis |
| Core Material Active agent | sodium sulfate | 31.0 |
| | Enzyme | 2.4 |
| | Cornstarch | 9.4 |
| 1st Coating | sodium sulfate | 43.7 |
| 2nd Coating | gum Arabic | 13.5 |

| Formulation #27 | | |
| --- | --- | --- |
| | Component | % (w/w) dry basis |

| Core Material Active agent | sodium sulfate | 31.0 |
|---|---|---|
| | Enzyme | 2.4 |
| | Cornstarch | 9.4 |
| 1st Coating | MgSO4 | 43.7 |
| 2nd Coating | Whey | 13.5 |

| Formulation 28** | | |
|---|---|---|
| | Component | % (w/w) Dry Basis |
| Core Material | sodium sulfate | 32.7 |
| Active Agent #1 | enzymes #1 | 9.1 |
| | sucrose | 4.1 |
| | corn starch | 8.5 |
| Active Agent #2** | enzyme #2 | 1.9 |
| | sucrose corn starch | 0.9 1.8 |
| Coating | sodium #1 sulfate | 33.6 |
| Coating #2 | PVA 51-05 | 2.5 |
| | talc | 5.0 |
| **Inherently thermostable enzyme | | |

| Formulation #29** | | |
|---|---|---|
| | Component | % (w/w) Dry Basis |
| Core Material | sodium sulfate | 32.7 |
| Active Agent and #2 Mixed | #1 enzyme #1 and #2 | 10.9 |
| | sucrose | 5.0 |
| | corn starch | 10.3 |
| Coating #1 | sodium sulfate | 33.6 |
| Coating #2 | PVA 51-05 | 2.5 |
| | talc | 5.0 |
| **Inherently thermostable enzyme | | |

| Formulation #30** | | |
|---|---|---|
| | Component | % (w/w) Dry Basis |
| Core Material | Sand | 84.80 |
| Active Agent | #1 enzyme #1 | 3.52 |
| | sucrose | 1.62 |
| | Whole Ground Wheat | 3.31 |
| Coating #1 | sodium sulfate | 5.50 |
| Coating #2 | PVA 51-05 | 0.42 |
| | talc | 0.83 |
| **Inherently thermostable enzyme | | |

| Formulation #31 | | |
|---|---|---|
| | component | % (w/w) Dry Basis |
| Core Material | sodium sulfate | 40.0% |
| Spray 1 | enzyme | 5.0% |
| | PVA | 1.0% |
| | corn starch | 5.0% |
| Spray 2 | sodium sulfate | 40.0% |

| Spray 3 | PVA | 3.0% |
|---|---|---|
| | talc | 6.0% |
| Formulation #32 | | |
| | Component | % (w/w) Dry Basis |
| Core Material | sodium sulfate | 40.0% |
| Spray 1 | enzyme | 5.0% |
| | corn starch | 9.0% |
| Spray 2 | sodium sulfate | 39.0% |
| Spray 3 | gum arabic | 7.0% |
| Formulation #33 | | |
| | Component | % (w/w) Dry Basis |
| Core Material | Sodium sulfate | 54.8 |
| Active agents | Mixed enzymes | 11.8 |
| (2) | sucrose | 5.4 |
| | corn starch | 11.1 |
| Coating 1 | Sodium sulfate | 26.0 |
| Coating 2 | PVA | 2.5 |
| | talc | 4.9 |
| Formulation #34 | | |
| | Component | % (w/w) Dry Basis |
| Core Material | Sodium sulfate | 54.8 |
| Active agents (2) | E enzyme mixture | 11.8 |
| Coating 1 | | 26.0 |
| Coating 2 | Sodium sulfate | 2.5 |
| | PVA | 4.9 |
| | talc | |

EXAMPLE 1. PREPARATION OF GRANULES IN TABLE 2.

All of the granules in Table 2, except granule number 1, are granules that were prepared using a fluid bed process as described in US Pat. No. 5,324,649. The fluid bed process fluidized the core materials in a Vector FL- 1 processor (made by Vector Corp, Marion, IA, USA), a Glatt 3, or a Uniglatt processor (both made by Glatt Air Techniques, Binzen, Germany). An enzyme/sugar/starch mixture was spray coated onto the core material. Then, any protective coating(s) were sprayed sequentially onto the enzyme layer and allowed to dry.
For example, the formulation #3 granule was prepared as follows:

In a Glatt 3 top spray fluid bed coater, sodium sulfate crystals screened to -45/+140 mesh were charged and fluidized using a heated bed temperature. A xylanase ultra filtration concentrate from Trichoderma reesei was mixed with corn starch and sucrose and sprayed onto the crystalss. The solution was about 33% dry solids. The final batch weight was 4000 grams.
In a vector FL-1 top spray coater, moisture hydrating material, a magnesium sulfate solution, was sprayed onto 833 grams of the material described above. The bed temperature was 50°. The final batch weighed 2500 grams.

Preparation of Granule number 1 further included a heat annealing step in which the granule was heated to a temperature sufficient to exceed the glass transition temperature (Tg) of the carnauba wax material followed by slow cooling which produced a hardened, glassy wax layer.

EXAMPLE 2: PREPARATION OF MASH SAMPLES WITH GRANULES AND PELLETING

Three different feed formulations and pelleting processes were used to prepare pellets with the granules listed in Table 2. Relatively high dosages of granules were added to the feed formulations to optimize the active agent remaining activity assays.

Mill #1:

Selected granules from Table 2 were mixed together with a feed formulation. The composition of the feed formulations was as follows:

75% (w/w) cornmeal (enriched yellow degerminated cornmeal, no 50956, General Mills Operations, Minneapolis, MN);and

25% (w/w) soybean meal (Pro Soybean meal, Cargill Oilseed Co., Cedar Rapids, IA).

12 kg of the above feed mixture was combined with each sample granule (dosed at 5 g/kg of feed formulation), and blended in a large Hobart blender (model D-300T, Troy OH), for 8 minutes. Approximately 150 g of each sample was retained as the mash sample, or unpelleted feed mixture. Each batch was then split into three 4 kg sub-batches, and pelleted in triplicate pellet mill runs.

The pellet mill used was CPM model CL5 (California Pellet Mill Co., Crawfordsville, IN). The temperature in the steam conditioner was controlled by the amount of injected steam, at 1.36 atm. Conditioning temperature of the mash, measured immediately prior to entering the die of the pellet mill, was targeted to 89-90°C. Temperature measurement was done using a thermometer and a "J" type thermocouple (OMEGA Engineering, Inc. Stamford, CT). The residence time in the conditioner was approximately 5 seconds. Die dimensions were 12.7 cm inner diameter, 17.8 cm outer diameter, 4.7 mm hole diameter. The pellet mill was run continuously, and the samples were processed through the mill sequentially, separated by running approximately 2 kg of a commeal/soy mix between samples. Approximately 1 minute after the test mash was added to the conditioner, and the conditioner temperature had stabilized to target temperature, pellets were collected over a 30 second interval, allowing approximately 1 kg of pellets to be collected for each sample. The collected pellets were held under ambient conditions, for 30 seconds, and then cooled with an air cooler for 2-3 min to room temperature.

Mill #2:

Prepared durable granules from Table 2 were pelleted with a corn and soy feed formulation. The exact composition of the feed formulations was as allows: 61.2% (w/w) cornmeal, 31.6% (w/w) soybean meal, 3.0% meat and bone meal, 2.5% soybean oil, 1.3% limestone, 0.28 % Salt, and 0.08% methionine. For each granule tested, 370 to 2000 grams of the granules, depending on enzyme type and activity, were combined with 450 kg of the feed without oil, and blended in a plow mixer for 2 minutes. Then soybean oil was added and the sample was mixed for an additional 3 minutes. The pellet mill was the Master model, manufactured by California Pellet Mill. Die pellet hole diameter was 4.5 mm. The typical feed rate was 780 kg per hour. The temperature in the steam conditioner was controlled manually, and was measured at the feed outlet from the conditioner. Two conditioning temperatures were used; 85°C and 95°C. The residence time in the conditioner was approximately 30 seconds. When the target temperature was reached, the system was run for approximately 5 minutes before sampling took place. Samples of approximately 5 kg of pelleted feed were taken, and were cooled by spreading on screened trays.

Mill #3:

The prepared durable granules were pelleted with a wheat and soy feed formulation or a wheat and barley formulation. The composition of the feed formulation was either 60 % wheat, 31.5% soybean meal, 4% soybean oil, 1.5% dicalcium phosphate, 1.23% vitamin mineral premix, 1.2% limestone, 0.4% salt, and 0.2% DL methoinine; or 60% wheat, 30% barley, with two conditioning temperatures, 90° C and 95° C used. For each granule tested, 200 to 500 grams of granules, depending on enzyme type and activity, was combined with 160 kg of the feed, and blended in a horizontal ribbon mixer, for approximately 15 minutes. The pellet mill was a Simon Heesen, mono roll type, fitted with a 17.3 cm inner diameter die, with a pellet hole diameter of 3 mm. Die speed was 500 rpm and was driven by a 7.5 kW motor. The typical feed rate was 300 kg per hour. The temperature in the steam conditioner was kept at +/- 0.1 degrees Celcius, measured at the feed outlet from the conditioner. The conditioner had a cascade type mixer system. Three conditioning temperatures were used; 85°C90 °C, and 95°C. Steam inlet pressure was 2 atm, and the temperature in the conditioner was controlled by manual adjustment of three valves that regulate the steam delivery. The residence time in the conditioner was approximately 30 seconds. When the target temperature was reached, the system was run for approximately 5 to 10 minutes before sampling took place. Samples were taken for 1-1.5 minute periods, corresponding to 5-7.5 kg of pelleted feed, and were immediately placed in a cooling box with a perforated bottom and air flow of 1500 cubic meters per hour. After cooling for 15 minutes, the samples were downsized twice using a sample divider, and 1 kg was taken for lab tests.

EXAMPLE 3: ENZYME ACTIVITY MEASUREMENTS

Determination of enzyme activity

To determine the enzyme activity after pelleting, the mash and pelleted samples were then ground for 30 seconds in a kitchen coffee grinder (model 203-42, Krups North America Inc., Medford MA), and assayed for enzyme activity as described below. Alternatively, samples were ground in a ZM-200 centrifugal mill, fitted with a 1 mm sieve (Retsch GmbH, Germany).

Calculation of Percent Recovered Activity:

For each test sample, both mash and the corresponding pelleted samples were assayed for activity. The percent recovered activity was calculated as follows:

$$\% \text{ recovered activity} = \frac{\text{activity in pellet} \times 100}{\text{activity in the mash}}$$

The phytase enzyme assay was conducted according to AOAC (Association of Analytical Chemists) Official Method 2000.12, as described in "Determination of phytase activity in feed by a colorimetric enzymatic method: collaborative interlaboratory study". Engelen AJ, van der Heeft FC, Randsdorp PH, Somers WA, Schaefer J, van der Vat BJ. J AOAC Int. 2001 May-Jun; 84(3):629-33. Briefly, the ground samples extracted in 220 mM sodium acetate trihydrate, 68.4 mM calcium chloride dihydrate, 0.01% Tween 20, pH 5.5. The supernatant is then assayed. The assay measures the release of inorganic phosphate from rice phytate, at pH 5.5, for 60 min at 37C. The assay is stopped with acidic molybdate/vanadate reagent, and phosphate is quantified by intensity of yellow colored complex of the vanadomolybdophosphor.

The xylanase enzyme assay was performed using the Xylanase Assay Kit (Xylazyme AX Format), Cat No: K-XYLS Megazyme International Ireland Ltd., Wicklow, Ireland. Materials for the assay include 16 x 125 mm disposable glass culture tubes; Extraction buffer: 100 mM MES sodium salt buffer, pH 6.0; Assay buffer: 25 mM sodium phosphate buffer, pH 6.0; Stopping solution: 20 g $NaPO_412H_20$ in 1 liter MilliQ water; Polystyrene Tissue Culture Flasks, 225 ml (Coming Incorporated - Life Sciences Big Flats, New York). 6-12 g of ground feed is extracted in 120 ml of extraction buffer, in a culture flask, for 1 hour at 20-25° C, while shaking on an orbital gel shaker set to 100 rpm. Samples are then centrifuged at 2000 g for 1 min, and the supernatant, is assayed. For the assay, 20 to 100 ul of the feed supernatant is diluted into 500 ul using assay buffer, in culture tubes, and equilibrated in a water bath at 40°C for 10 min. Then, one substrate tablet is placed into each culture tube, and samples are incubated for an additional 10 minutes at 40C. 10 ml of stopping solution is then added to each tube. The samples are vortexed briefly, and then filtered through Whatman #1 filter paper. The absorbance of the filtrate is read with a spectrophotometer at a wavelength of 590 nm. The spectrophotometer is first zeroed with a blank, made by combining 20 to 100 ul of the feed supernatant, 500 ul assay buffer, and 10 ml of stopping solution. One substrate tablet, is then added, followed by, vortexing, and filtering in the same manner as the samples. Interference from some feed components may affect assay response. In order to correct for any interference, standard curves with feed background were prepared. Uncoated xylanase granules were added to blank mash, and to blank pellets, at several levels. The spiked mash and pellet samples were then ground, and extracted exactly as described above. From this series of extracts, standard curves for both the mash and the pellets were generated.

The beta-glucanase enzyme assay was performed using a Beta-glucanase Assay Kit (Beta-Glucazyme Tablet format). Cat No: T-BGZ200, Megazyme International Ireland Ltd., Wicklow, Ireland. Materials for the assay includes 16 x 125 mm disposable glass culture tubes; Extraction buffer and assay buffer: 25 mM sodium acetate buffer; Stopping solution 2% $NaPO_412H_20$ in 1 liter MilliQ water; 140 ml glass beakers. 10 g of ground feed was extracted in 100 ml of extraction buffer, by mixing in a beaker for 1 hour at 20-25°C. Samples are then centrifuged at 2000 g for 1 min. and the supernatant is assayed. For the assay, 20 to 100 ul of the feed supernatant is diluted into 500 ul using assay buffer, in culture tubes, and equilibrated in a water bath at 40°C for. 10 min. Then, one substrate tablet is placed into each culture tube, and samples are incubated for an additional 10 minutes at 40°C. 10 ml of stopping solution is then added to each tube. The samples are vortexed briefly, and then filtered through Whatman #1 filter paper. The absorbance of the filtrate is read with a spectrophotometer at a wavelength of 590 mn. The spectrophotometer is first zeroed with a blank, made by combining 20 to 100 ul of the feed supernatant, 500 ul assay buffer, and 10 ml of stopping solution. One substrate tablet, is then added, followed by, vortexing, and filtering in the same manner as the samples. A standard curve is prepared by assaying a series of enzyme diluted to appropriate levels in assay buffer.

RETAINED ENZYME ACTIVITY RESULTS

Table 3: Pelleting Results - Percent Recovered Activity after pelleting

| Formulation | Mill #1, 90°C | Mill #2,3, or 4 at 85-95°C |
|---|---|---|
| 10 | <30% xylanase | |
| 12 | <30% xylanase | |
| 13 | <30% xylanase | |
| 14 | <30% xylanase | |
| 15 | <30% xylanase | |
| 16 | <30% xylanase | |
| 21 | <30% xylanase | |
| 25 | <30% xylanase | |
| 1 | >50% xylanase | |
| 2 | >50% xylanase | |
| 3 | >70% xylanase | >70% xylanase |
| 5 | >50% xylanase, >70% e.coli phytase | >50% xylanase, >80% e. coli phytase |
| 6 | >50% xylanase | |
| 7 | >50% xylanase | |
| 9 | >50% xylanase | |
| 11 | | >50% xylanase, >70% Aspergillus phytase |
| 30 | | >60% thermostable xylanase |
| 22 | >50% xylanase | |
| 23 | >60% e. coli phytase | >50% xylanase, >80% e. coli phytase |
| 27 | >50% xylanase | >80% e. coli phytase |
| 33 | | >90% BGL, >70% thermostable xylanase |
| 4 | >70% xylanase | |
| 8 | >70% xylanase | |
| 17 | >70% xylanase | |
| 18 | >70% xylanase | |
| 19 | >70% xylanase | |
| 20 | >70% xylanase | |
| 26 | >70% xylanase | >90% e. coli phytase |
| 28 | | >90% BGL, >90% thermostable xylanase |
| 29 | | >70% BGL, >90% thermostable xylanase |
| 31 | | >90% e. coli phytase |
| 32 | | >90% e. coli phytase |

The pellet mills used can be broken into two groups. Mill #1 is a lab scale mill, which is much harsher than what is typically encountered in commercial practice. The harshness is attributed to the large amount of steam which is delivered to a relatively small portion of mash, coupled with the fact that the feed mixture does not contain oil, which typically lubricates the feed while traveling through the die. Mills #2 through #4 are considered to be more representative of commercial conditions.

The retained activities of the tested granules are shown in Table 3. Granules of the present invention that are not considered stable, durable granules were numbers 10, 12, 13, 14, 15, 16,21 and 25, all of which demonstrated less than 30% recovered activity. These granules have a core coated with an enzyme matrix layer wherein the coated core is greater that about 50 w/w% of the entire granule. Most of these non-stable granules had only one protective coating layer of a moisture barrier material, such as carnauba wax, or ethylcellulose, or HPMC, or canola oil. Without wishing to be bound by any particular theory, and recognizing that these granules may show greater than 50% retained active agent activity at 70-85°C, the poor thermostability of these granules under these test conditions, relative to stable, durable granules, may be due to the provision of coating materials as relatively thin layers, comprising, in five examples, about 2.0 to about 17.0% w/w of the granule, and/or the particular coating agent employed. For instance, granule number 10 has a single layer of coating material that is 50 w/w% moisture barrier material (camauba wax); and granule number 25 has two moisture barrier protective coating layers that together constitute 32% w/w of the granule.

Particularly stable, durable granules of the present invention were numbers 4, 8, 17, 18, 19, 20, 26, 28, 29, 31 and 32, all of which have greater than 70% Recovered xylanase activity when pelleted at about 90° C for about 5 seconds. Additionally, granules 26, 31 and 32 demonstrated greater than 90% recovered phytase activity when pelleted between 85° C and 95° C for about 30 seconds. Granules 28 and 29 demonstrated greater than 90% retained activity of one inherently thermostable xylanase in the enzyme mixture and 70%-90% retained activity of a thermotabile beta-glucanase when pelleted between 85° C and 95° C for 30 seconds. Those stable durable granules having an active agent that is not inherently thermostable are either; 1) coated with a single, thick protective layer of a moisture hydrating material, or 2) coated with two to three protective layers, where at least one of the layers is a moisture hydrating material and one layer is a moisture barrier material.

In general, when moisture hydrating materials are applied as individual layers, the coating must comprise from about at least about 55% of the granule, to provide suitable thermo protection; and, when the amount of moisture hydrating material was lowered to less than 55% using an individual layer, percent retained activity dropped to less than 50%. However, when moisture hydrating materials are used in combination with moisture barrier materials, the water hydrating protective layer may be lowered to at least about 25% w/w of the granule, while the moisture barrier material may be about 2% to 40% w/w of the granule.

Other stable, durable granules of the present invention were numbers 1, 2, 3, 5, 6, 7, 9, 11, 22, 23, 27, 30 and 33 all of which demonstrated greater than 50% recovered xylanase or phytase activity when pelleted at 90°C for 5 seconds or at 85° C to 95° C for 30 seconds. As can be seen from these results, inorganic salt outer or inner coatings alone provide good protection for the enzyme and enhanced protection when combined with moisture barrier coatings. As noted earlier, granule number 1 was made using a heat annealing processing step and it is believed that addition of a heat annealing step enhances barrier properties of the moisture barrier materials, such as polymers, proteins, and lipids thereby reducing the amount of moisture hydrating material needed to provide thermostability. When a granule otherwise identical to granule #1 was made without heat annealing, the recovered activity was 35%. Granules 2, 3, and 22 show that different core materials can be used without compromising granule stability.

Granule 3 demonstrated greater than 70% recovered enzyme activity at both 5 seconds and 30 seconds pelleting time periods. Granule 11 demonstrated greater than 50% recovered xylanase activity when pelleted at 85° C to 95° C for about 30 seconds, and greater than 70% recovered phytase activity when pelleted at 85° C to 95° C for about 30 seconds. Granule #23 demonstrated greater than 60% recovered activity of phytase when pelleted for 5 seconds at 90° C; greater than 50% recovered activity of xylanase when pelleted at 85° C to 95° C for 30 seconds; and, greater than 80% recovered activity of phytase when pelleted at 85° C to 95° C for 30 seconds.

EXAMPLE 4: DIRECT STEAMING TEST

100 gram portions of mash comprised of 75% cornmeal and 25% soy were dosed with granules, containing *E. coli* phytase, at an inclusion rate of 1.25 g of granules per 1 kilogram of mash. Mash samples were then wrapped in a cheese cloth bag, and steamed for 20 seconds by placing the bags into a funnel with 30 psi steam applied to the bottom of the funnel. After steaming, samples were cooled to ambient temperature, allowed to dry overnight, and then assayed for phytase activity. The percent recovered activity of the steamed samples, relative to unheated mash, was reported.

A granule that was not considered suitably steam resistant under these test conditions was number # 25, which had less than 35% recovered activity after steaming. Stable, durable, granules of the present invention were numbers #5 and #23, which had greater than 50 % recovered activity after steaming.

EXAMPLE 5: BIOAVAILABILITY TEST OF PELLETED GRANULES

The stable, durable granules of the present invention may be tested for bioavailability of the active agent enzyme using known bioavailability tests, such as the phytase bioavailability study disclosed in WO 00/47060; bioavailability tests described in Enzymes in Animal Nutrition, Proceedings of the 1st Symposium, Kartause Ittingen, Switzerland, October 13016, 1993; the bioavailability tests described in Chemgen patents.

Stable granules of the present invention were assessed after pelleting to determine bioavailability in broiler chicks fed a commercial diet.

Materials and Methods

Tested Enzymes and Granules and Dietary Treatments included Phyzyme® XP (6-phytase, E.C. 3.1.3.26) enzyme alone, and both PVA-coated and Gum Arabic (GA) coated stable granules of the present invention containing Phyzyme XP enzyme. (See Table 4). The tested enzymes and granules were fed to the broiler chickens in either a mash diet or a pellet (prepared at 90°C) diet. The experimental diets were: positive control (commercial diet), negative control, negative control + 500 U/kg Phyzyme® XP, negative control + 500 U/kg PVA-coated-phytase, negative control + 500 U/kg GA-coated-phytase, negative control + 500 U/kg PVA-coated-phytase pelleted at 90°C, and negative control + 500 U/kg GA-coated-phytase pelleted at 90° C. Except for calcium and phosphorus, all the diets were isocaloric and isonitrogenous. (See Table 6). Calcium and available phosphorus in the positive and negative control diets were 0.90% and 0.78%, and 0.37% and 0.26%, respectively. Titanium dioxide (0.10%) was added to the diets as an indigestible marker to help estimate nutrient digestibility. The experimental diets were manufactured by ADAS Gleadthorpe to a commercial specification and retained at ADAS Gleadthorpe, and stored under refrigeration. Samples of the enzyme additive mixes (25g), cereal (250g), soybean meal (250g) and a sample of all the diets (250g) were sent to Danisco Animal Nutrition Enzymes Feed Services, Edwin Rahrs Vej 38, DK-8220 Brabrand, Denmark. All feed samples were assayed for phytase prior to feeding.

Experimental Animals and Design:

The chicks were male Ross 308 broilers aged 0 to 21 days using eight replicate pens per treatment with 30 birds per pen. The chicks were placed randomly into boxes before being weighed and randomly allocated to the treatment pens. Each pen contained one tube feeder and birds had free access to water via nipple drinkers (4 per pen) and feed at all times. The height of the drinkers was adjusted regularly to keep level with the top of the back of the birds. Litter was provided in the form of clean wood shavings to a depth of 5cm. The house was heated by a warm air brooding system and the brooding target temperature regime was 31°C at day old, reducing by 1°C every other day until 21°C was reached on day 21. The minimum ventilation rate was automatically calculated to supply $1.9 \times 10^4 m^3$ of air per second per $kg^{0.75}$ bodyweight and this rate was supplied by one 610mm fan controlled by a Farm-Ex Diacam control panel. Lighting programme was 23 hours light and 1 hour dark. Light intensity was reduced from maximum attainable at day old (40-60 lux) to an intensity of approximately 10 lux by 10 days of age. Relative humidity was monitored on a daily basis using a Tinytalk© data logger. Chicks were vaccinated with IB H120 & 50% Avinue (ND) at Hatchery.

Feed Intake/Body Weight

Feed usage was measured between 0 and 21 days. The feed in each pen was measured by weighing back the amount of feed remaining at the end of the period and deducting it from the quantity offered. The birds were weighed in their pen groups at day old. At 21 days all the birds in all of the pens were weighed in batches. The total weight of all the birds in each pen was determined and the mean calculated. All birds that died were weighed and the details recorded, and any lame birds or birds unable to reach feed and water were culled from the study and the reason for culling was listed.

Tibia ash

At 21 days of age 2 birds from each pen were culled using cervical dislocation and the left leg removed. The tibia was dissected out and the bone sent to Eurofins laboratories, Woodthorne, Wolverhampton for ashing on a plot basis. All the data were analyzed using general linear models procedure of SAS (SAS Inst. Inc., Cary, NC). Where significant differences were found, Duncan's test was used to compare individual treatments means.

Results and Discussion

Analyzed phytase activity in the experimental diets were <50, <50, 563, 467, 558 FTU/kg (PVA-coated-phytase), and 554 and 440 (GA-coated-phytase), for positive control, negative control, negative control + 500 U/kg Phyzyme® XP, negative control + 500 U/kg PVA-coated-phytase and GA-coated-phytase, and negative control + 500 U/kg PVA-coated-phytase and GA-coated-phytase pelleted at 90°C, respectively (Table 5), indicating that activity of both the PVA-coated-phytase and the GA-coated-phytase was not destroyed after pelleting at 90°C,

Birds fed diets supplemented with 500 U/kg PVA-coated-phytase and GA-coated-phytase, pelleted at 90°C, were heavier at day 21 than birds fed the positive control, negative control and negative control + 500 U/kg Phyzyme® XP diets (Table 7). Birds fed negative control diet consumed less feed than birds fed diet supplemented with 500 U/kg PVA-coated-phytase and GA-coated-phytase, pelleted at 90°c (Table 8). Birds fed the mash diets supplemented with 500 U/kg.Phyzyme® and PVA-coated-phytase and GA-coated-phytase showed no statistical difference with the positive control. Those fed diets supplemented with 500 U/kg PVA-coated-phytase and GA-coated-phytase, pelleted at 90°C, had better feed conversion ratio at day 21 than birds fed negative control + 500 U/kg Phyzyme® XP and positive control diets (Table 9). Tibia ash was lowest for birds fed negative control diet compared to all other treatments. Birds fed positive control and phytase-supplemented diets had similar tibia ash (Table 10).

The phytase tested, either as Phyzyme® XP or coated with PVA or GA, performed as well as the positive control diet when fed as mash. When PVA or GA-coated phytase was added to commercial diets pelleted at 90°C. recovery of the product was maintain at non-pelleted levels and the performance was at least as good as or better than the positive control diet. The results indicate that coating enzyme with PVA or GA improved its thermostability at pelleting temperature of 90°c without losing bioefficacy in broilers fed commercial diets.

Table 4 Dietary treatments

| Dietary treatment | Enzyme, U/Kg | Inclusion(g/T) |
|---|---|---|
| Positive control | 0, mash diet | 0 |
| Negative control | 0, mash diet | 0 |
| Phyzyme XP Mash diet | 500 U/kg Phyzyme XP, mash diet | 100 |
| PVA-coated Mash diet | 500 U/kg PVA-coated phytase, mash diet | 100 |
| PVA coated pellet diet | 500 U/kg PVA-coated phytase (Pellet at 90°C) | 100 |
| GA-coated Mash diet | 500 U/kg GA-coated phytase, mash diet | 100 |
| GA coated pellet diet | 500 U/kg GA-coated phytase (Pellet at 90°C) | 100 |

Table 5 Phytase activity in experimental diets

| Dietary treatment | Expected | Observed | Observed, % |
|---|---|---|---|
| | FTU/Kg | | |
| Positive control, Mash | <50 | <50 | - |
| Negative control, Mash | <50 | <50 | - |
| Phyzyme XP, Mash diet | 500 | 563 | 112.6 |
| PVA-coated, Mash diet | 500 | 467 | 93.4 |
| PVA coated pelleted at 90°C | 500 | 558 | 111.6 |
| GA-coated, Mash diet | | | |
| GA coated Pelleted at 90° C | 500 | 440 | 88.0 |

Table 6 Experimental Diets

| Ingredients: % | Positive control | Negative control |
|---|---|---|
| Maize | 58.56 | 59.49 |
| Soybean meal -48 | 34.65 | 34.55 |
| Soya oil | 2.82 | 2.48 |

(continued)

| Ingredients: % | Positive control | Negative control |
|---|---|---|
| salt | 0.30 | 0.30 |
| Sodium Bicarbonate | 0.20 | 0.20 |
| Dicalcium Phos. | 1.59 | 0.83 |
| Limestone | 0.95 | 1.12 |
| Vitamin premix | 0.50 | 0.50 |
| Lysine-HCl | 0.10 | 0.10 |
| DL-Methionine | 0.23 | 0.23 |
| Titanium dioxide | 0.10 | 0.10 |
| Enzyme premix carrier (corn) | 0 | 0.10 |
| *Nutrient Composition* | | |
| Crude Protein, % | 21.68 | 21.68 |
| Poultry metabolisable energy, MJ/Kg | 12.8 | 12.8 |
| Calcium, % | 0.90 | 0.78 |
| Phosphorus, % | 0.67 | 0.54 |
| Available phosphorus, % | 0.37 | 0.26 |
| Methionine+Cystine, % | 0.92 | 0.92 |
| Methionine, % | 0.56 | 0.56 |
| Lysine, % | 1.25 | 1.25 |
| Threonine, % | 0.82 | 0.82 |
| Tryptophan, % | 0.25 | 0.25 |

Table 7 Initial and Final Bodyweight of Broilers fed Experimental Diets

| Dietary treatment | Bodyweight, g | |
|---|---|---|
| | Day 0 | Day 21 |
| Positive control, Mash | 44.43 | 789[b] |
| Negative control, Mash | 43.55 | 680[d] |
| 500 U/kg Phyzyme XP, Mash | 43.72 | 753[c] |
| 500 U/kg PVA-coated phytase, Mash | 43.75 | 740[c] |
| 500 U/kg GA-coated phytase, Mash | | |
| 500 U/kg PVA-coated phytase, Pelleted at 90 °C | 43.70 | 899[a] |
| 500 U/kg GA-coated phytase, Pelleted at 90 °C | 43.67 | 934[a] |
| Standard error of difference between means | 0.261 (PVA) 0.228 (GA) | 6.015 (PVA) 7.741 (GA) |
| P value | 0.165 (PVA) 0.053 (GA) | <0.001 (PVA) <0.001 (GA) |
| Differing superscripts in the same column indicate significant difference | | |

Table 8 Overall Feed Intake of Broilers fed Experimental Diets

| Dietary treatment | Feed intake (g/bird/day) |
|---|---|
| Positive control, Mash | 56.69[b] |
| Negative control, Mash | 46.00[a] |
| 500 U/kg Phyzyme XP, Mash | 59.03[b] |
| 500 U/kg PVA-coated phytase, Mash | 56.70[b] |
| 500 U/kg PVA-coated phytase, Pelleted at 90 °C | 57.18[b] |
| 500 U/kg GA-coated phytase, Pelleted at 90° C | 64.33[b] |
| Standard error of difference between means | 2.321 (PVA) <br> 2.260 (GA) |
| P value | 0.004 (PVA) <br> 0.001 (GA) |
| Differing superscripts in the same column indicate significant difference | |

Table 9 Feed Conversion Ratio of Broilers fed Experimental Diets

| Dietary treatment | Feed Conversion Ratio |
|---|---|
| Positive control, Mash | 1.617[ab] |
| Negative control, Mash | 1.548[ab] |
| 500 U/kg Phyzyme XP, Mash | 1.752[a] |
| 500 U/kg PVA-coated phytase, Mash | 1.715[a] |
| 500 U/kg PVA-coated phytase, Pelleted at 90 °C | 1.412[b] |
| 500 U/kg GA-coated phytase, Pelleted at 90° C | 1.537 |
| Standard error of difference between means | 0.715(PVA) <br> 0.069 (GA) |
| P value | 0.016 (PVA) <br> 0.061 (GA) |
| Differing superscripts in the same column indicate significant difference | |

Table 10 Tibia Ash Content of Broilers fed Experimental Diets

| Dietary treatment | Tibia ash content (g/100g) |
|---|---|
| Positive control, Mash | 14.03[b] |
| Negative control, Mash | 10.86[a] |
| 500 U/kg Phyzyme XP, Mash | 13.93[b] |
| 500 U/kg PVA-coated phytase, Mash | 13.24[b] |
| 500 U/kg PVA-coated phytase, pelleted at 90 °C | 13.50[b] |
| 500 U/kg GA-coated phytase, pelleted at 90° C | 13.84[a] |
| Standard error of difference between means | 0.403 (PVA) <br> 0.302 (GA) |
| P value | <0.001 (PVA) |

(continued)

| Dietary treatment | Tibia ash content (g/100g) |
|---|---|
|  | <0.001 (GA) |
| Differing superscripts in the same column indicate significant difference |  |

EXAMPLE 6: ENZYME PREMIX & PELLETING STABILITY

Granules containing phytase, made according to formulations in Table 2 having either a PVA or a gum arabic coating and a moisture hydrating coatings, were mixed with the clay sepiolite, or a standard broiler vitamin mineral premix with and without choline chloride. Blending ratios were 100 grams of granules added to 900 grams of clay (sepiolite), or 100 grams of granules added to 500 grams of vitamin mineral premix. Samples were stored in sealed containers at 35°C for 3 weeks, and then subjected to pelleting, as described above in Mill #3. The experimental control was granules that were not stored in a premix, and were held at ambient conditions for 3 weeks.

Tables 11 and 12 show the percent-recovered phytase activity of these mixtures after pelleting at 90°C and 95°C. Granules mixed with sepiolite or the vitamin mineral premixes were found to have significantly increased recovered activity after pelleting.

| Table 11 |  |  |  |  |
|---|---|---|---|---|
| PVA Formulation Granules |  |  |  |  |
| Premix | Percent recovered activity after pelleting, relative to mash | | Percent increase in recovered activity, relative to control granules | |
|  | 90°C | 95°C | 90°C | 95°C |
| granules alone (control) | 71% | 57% |  |  |
| Sepiolite + granules | 95% | 88% | 34% | 53% |
| Vitamin mineral premix, no choline chloride + granules | 93% | 80% | 32% | 40% |
| Vitamin mineral premix, with choline chloride + granules | 98% | 85% | 39% | 48% |

| Table 12 |  |  |  |  |
|---|---|---|---|---|
| GA Formulation Granules |  |  |  |  |
| Premix | Percent recovered activity after pelleting, relative to mash | | Percent increase in recovered activity, relative to control granules | |
|  | 90°C | 95°C | 90°C | 95°C |
| granules alone (control) | 92% | 81% |  |  |
| Sepiolite + granules | 99% | 88% | 8% | 9% |
| Vitamin mineral premix, no choline chloride + granules | 100% | 81% | 8% | 0% |
| Vitamin mineral premix, with choline chloride + granules | 97% | 80% | 5% | -2% |

Without wishing to be bound by any particular theory, it is believed that the clay and the vitamin mineral premix have a capacity to take up water and, during storage with stable granules, they absorb residual moisture from the granules. To illustrate this effect, Table 13 shows the results of an experiment in which granules and sepiolite are stored in open containers, positioned side-by-side, within a closed chamber. The water activity of the granules, and of the sepiolite, was measured before and after 7 days of storage at 25 °C. During storage, the sepiolite absorbed water from the granules until the system reached an equilibrium. After storage, the granules show a decrease in water activity, while the sepiolite shows an increase in water activity.

| Table 13 | | | | |
|---|---|---|---|---|
| | Initial water activity | | Water activity after 7 days of storage | |
| | Sepiolite | granules | Sepiolite | granules |
| Formulation GA granule | 0.312 | 0.558 | 0.359 | 0.373 |
| Formulation PVA granule | 0.312 | 0.516 | 0.354 | 0.365 |

EXAMPLE 7: STORAGE STABILITY WITH CHOLINE CHLORIDE

Choline chloride, or N-(2-Hydroxyethyl) trimethylammonium chloride, is an important feed additive, a vitamin nutrient, in poultry, pig and other animal feeds. Choline chloride is a reactive molecule and has a well-known destructive effect on other vitamins, and enzymes. Choline chloride is often included in premixes and base mixes. Maximum levels used in premixes are 74,800 mg/kg for swine, and 150,000 mg/kg for poultry. And typical levels for swine base mixes are about 966 to 1282.9 mg/kg.

The stable, durable granules of the present invention were shown to preserve enzyme activity when stored in the presence of choline chloride. Granules containing phytase, made with formulations with either PVA or gum arabic and a moisture hydrating material were mixed with a standard broiler vitamin mineral premix, with and without choline chloride. The blending ratio was 100 grams granules added to 500 grams of vitamin mineral premix. Samples were stored in sealed containers, at 35 °C, for 3 weeks, and then assayed for activity, using the phytase assay protocol described above. The experiment control was granules that were not stored in a premix, and were held at 35 °C for 3 weeks. Tables 14 and 15 show the measured activity of the mixtures before and after storage, and the percent change in activity. None of the samples show an appreciable loss of activity after storage. The error of this assay, including error of sampling, extraction, and activity assay is approximately 15%.

| Table 14 | | | |
|---|---|---|---|
| PVA Formulation Granule | | | |
| Sample | Initial activity (FTU/g) | Activity after storage 3 weeks at 35 °C (FTU/g) | percent change in activity |
| granules only (control) | 11,600 | 11,858 | 2% |
| granules + vitamin mineral premix without choline chloride | 1,933 | 1,727 | -12% |
| granules + vitamin mineral premix with choline chloride | 1,933 | 1,720 | -12% |

| Table 15 | | | |
|---|---|---|---|
| GA Formulation Granule | | | |
| Sample | Initial activity (FTU/g) | Activity after storage 3 weeks at 35 °C (FTU/g) | percent change in activity |
| granules only (control) | 10,373 | 10,528 | 1% |

(continued)

| Table 15 | | | |
| --- | --- | --- | --- |
| GA Formulation Granule | | | |
| Sample | Initial activity (FTU/g) | Activity after storage 3 weeks at 35 °C (FTU/g) | percent change in activity |
| granules + vitamin mineral premix without choline chloride | 1,729 | 1,543 | -12% |
| granules + vitamin mineral premix with choline chloride | 1,729 | 1,622 | -7% |

EXAMPLE 8: WATER ACTIVITY EFFECT ON PELLETING STABILITY

Phytase granules were prepared according to a formulation having a PVA coating and/or an inorganic salt layer coating, in a fluid bed process, as described above. An additional drying step can be used if the water activity of the granule is greater than 0.5 after processing, so that in a fluid bed coater, or other suitable process; the granules may be dried until a water activity of <0.5 is achieved. The results are shown in Table 16 and demonstrate that retained activity after pelleting is enhanced when the water activity is less than 0.5.

| Table 16 | | | | | |
| --- | --- | --- | --- | --- | --- |
| PVA Formulation Granule | | | | | |
| Granule | Water activity | Percent recovered activity after pelleting, relative to mash | | Percent increase in recovered activity, relative to control granules | |
| | | 90°C | 95°C | 90°C | 95°C |
| A (control) | 0.57 | 81% | 69% | | |
| B | 0.41 | 98% | 87% | 21% | 26% |
| C | 0.49 | 110% | 97% | 36% | 41% |

Embodiments:

1. A granule for feed compositions comprising:

a core;
an active agent; and
at least one coating, the active agent of the granule retaining at least 50% activity, at least 60% activity, at least 70% activity, at least 80% activity after conditions selected from one or more of a) a feed pelleting process, b) a steam-heated feed pretreatment process, c) storage, d) storage as an ingredient in an unpelleted mixture, and e) storage as an ingredient in a feed base mix or a feed premix comprising at least one compound selected from trace minerals, organic acids, reducing sugars, vitamins, choline chloride, and compounds which result in an acidic or a basic feed base mix or feed premix.

2. The granule of embodiment 1 wherein the at least one coating comprises a moisture hydrating material that constitutes at least 55% w/w of the granule.

3. The granule of embodiment 1 wherein the at least one coating comprises two coatings.

4. The granule of embodiment 3 wherein the two coatings are a moisture hydrating coating and a moisture barrier coating.

5. The granule of embodiment 4 wherein the moisture hydrating coating is between 25% and 60% w/w of the granule and the moisture barrier coating is between 2% and 15% w/w of the granule.

6. The granule of embodiment 5 wherein the moisture hydrating coating is selected from inorganic salts, sucrose, starch, and maltodextrin and the moisture barrier coating is selected from polymers, gums, whey and starch.

7. The granule of embodiment 1 wherein the active agent is one or more enzymes.

8. The granule of embodiment 1 wherein the feed pelleting process and the feed pretreatment process are conducted between 70° C and 95 C for up to several minutes.

9. The granule of embodiment 1 wherein the feed pelleting process and the feed pretreatment process are conducted between 85°C and 95°C.

10. The granule of embodiment 1 wherein the unpelleted mixture is a diluent selected from one or more of clays, wheat middlings or rice bran.

11. The granule of embodiment 1 wherein the feed base mix or feed premix comprises choline chloride and the active agent is at least one enzyme which retains at least 80% activity when stored in the feed base mix or feed premix.

12. An animal feed composition comprising the granule of embodiment 1.

13. The animal feed composition of embodiment 12 selected from an animal feed diluent, an animal feed base mix, an animal feed premix, mash, and an animal feed pellet.

14. A granule for animal feed comprising:

   a core;
   an active agent, the active agent of the granule retaining at least 80% activity after storage and after a steam-heated pelleting process where the granule is an ingredient;
   a moisture barrier coating; and
   a moisture hydrating coating that is at least 25% w/w of the granule, the granule having a water activity of less than 0.5 prior to the steam-heated pelleting process.

15. The granule of embodiment 14 wherein the moisture barrier coating is selected from polymers and gums and the moisture hydrating material is an inorganic salt.

16. The granule of embodiment 14 wherein the moisture hydrating coating is between 25% and 45% w/w of the granule and the moisture barrier coating is between 2% and 10% w/w of the granule.

17. The granule of embodiment 14 wherein the active agent is one or more enzymes.

18. The granule of embodiment 14 wherein the steam-heated pelleting process is conducted between 85° C and 95° C for up to several minutes.

19. The granule of embodiment 14 wherein the at least 80% activity is retained after storage of the granule in an unpelleted mixture comprising at least one compound selected from trace minerals, organic acids, reducing sugars, vitamins, choline chloride, and compounds which result in an acidic or a basic unpelleted mixture.

20. An animal feed composition comprising the granule of embodiment 14.

21. The animal feed composition of embodiment 20 selected from an animal feed diluent, an animal feed base mix, an animal feed premix, an animal feed mixture, and an animal feed pellet.

22. An animal feed ingredient comprising:

   a granule comprising a core; an active agent surrounding the core; and at least one coating surrounding the active agent, the active agent retaining at least 50% activity, at least 60% activity, at least 70% activity, at least 80% activity after conditions selected from one or more of a) a feed pelleting process, b) a steam-heated feed pretreatment process, c) storage, d) storage as an ingredient in an unpelleted mixture, and e) storage as an ingredient in a feed base mix or a feed premix comprising at least one compound selected from trace minerals, organic acids, reducing sugars, vitamins, choline chloride, and compounds which result in an acidic or a basic feed base mix or feed premix.

23. The animal feed ingredient of embodiment 22 wherein the at least one coating comprises two coatings.

24. The animal feed ingredient of embodiment 23 wherein the two coatings are a moisture hydrating coating and a moisture barrier coating.

25. The animal feed ingredient of embodiment 24 wherein the moisture hydrating coating comprises a material selected from inorganic salts, carbohydrates, maltodextrin and starch and the moisture barrier coating comprises a material selected from polymers, proteins, lipids, fats, oils, fatty acids, gums, starches, lecithin, and wax.

26. The animal feed ingredient of embodiment 22 wherein the core and the active agent together comprise at least 50% w/w of the granule.

27. The animal feed ingredient of embodiment 22 wherein the core and the active agent together comprise about 50% to about 60% w/w of the granule.

28. The animal feed ingredient of embodiment 22 wherein the core and the active agent comprise about 90% to about 100% w/w of the granule and the active agent is an inherently thermostable enzyme.

29. The animal feed ingredient of embodiment 22 wherein the active agent is thermolabile.

30. The animal feed ingredient of embodiment 22 further comprising a feed composition, the granule being dispersed in the feed composition.

31. The animal feed ingredient of embodiment 30 wherein the feed composition is a feed pellet, the active agent dispersed in the feed pellet having a retained activity that is at least 50%, at least 60%, at least 70%, at least 80%, and at least 90% of a pre-pelleting activity.

32. The animal feed ingredient of embodiment 30 wherein the feed composition is selected from clay, essential nutrients, grains, wheat, rye, rice and mixtures thereof

33. A process for producing a granule comprising an active agent for feed, the process comprising: preparing stable granules having a core, at least one active agent, and at least one coating; mixing the stable granules together with one or more of a) a diluent, b) a base mix, c) a premix, and d) a feed mixture for pelleting.

34. The process of embodiment 33 further comprising the step of pelleting the feed mixture for pelleting at a temperature of 70° C to 95° C for up to several minutes.

35. The process of embodiment 33 wherein the stable granules are mixed together with a clay.

36. The process of embodiment 33 wherein the stable granules are mixed together with a premix comprising choline chloride.

37. An animal feed composition made by the process of embodiment 33.

38. A process for making a stable granule comprising enzyme for storage in a feed premix comprising choline chloride, the process comprising:

providing a core material and enzyme, the enzyme distributed throughout the core material or layered over the core material;
applying to the core material and enzyme a moisture hydrating material to form a layer that is at least 25% w/w of the stable granule;
coating the layer with a moisture barrier material to form a coating that is at least 5% w/w of the granule, the applying and coating under conditions selected so that a water activity of the stable granule is less than 0.5.

39. The process of embodiment 38 wherein the moisture hydrating material is an inorganic salt and the layer is about 35% to about 45% w/w of the stable granule.

40. The process of embodiment 38 wherein the coating is selected from polymers and gums.

**Claims**

1. A granule for feed compositions comprising:

   a core;
   an active agent; and
   at least one coating, the active agent of the granule retaining at least 50% activity, at least 60% activity, at least 70% activity, at least 80% activity after conditions selected from one or more of a) a feed pelleting process, b) a steam-heated feed pretreatment process, c) storage, d) storage as an ingredient in an unpelleted mixture, and e) storage as an ingredient in a feed base mix or a feed premix comprising at least one compound selected from trace minerals, organic acids, reducing sugars, vitamins, choline chloride, and compounds which result in an acidic or a basic feed base mix or feed premix;
   wherein the feed base mix or feed premix comprises choline chloride and the active agent is at least one enzyme which retains at least 80% activity when stored in the feed base mix or feed premix.

2. The granule of claim 1 wherein the at least one coating comprises a moisture hydrating material.

3. The granule of claim 1 wherein the moisture hydrating material constitutes at least 55% w/w of the granule.

4. The granule of claim 1-3 wherein the at least one coating is a moisture hydrating coating and a moisture barrier coating.

5. The granule of claim 4 wherein the moisture hydrating coating is between 25% and 60% w/w of the granule and the moisture barrier coating is between 2% and 15% w/w of the granule.

6. The granule of claim 1 wherein the moisture hydrating coating is selected from inorganic salts, sucrose, starch, and maltodextrin.

7. The granule of claim 6 wherein the moisture hydrating coating is sodium sulfate.

8. The granule of claim 1 wherein the unpelleted mixture is a diluent selected from one or more of clays, wheat middlings or rice bran.

9. The granule of claim 1 wherein the active agent is a phytase from Citrobacter.

10. The granule of claim 1 having a water activity of less than 0.5.

11. An animal feed composition comprising the granule of any of claims 1-10.

12. The animal feed composition of claim 11 selected from an animal feed diluent, an animal feed base mix, an animal feed premix, mash, and an animal feed pellet.

13. The use of an animal feed composition according to claims 11 or 12 in a steam-treating and/or pelleting process.

14. The use according to claim 13 wherein the steam treating and feed pelleting process is conducted between 70°C and 95°C for up to several minutes.

15. The use according to claim 13 wherein the steam treating and feed pelleting process is conducted between 85°C and 95°C.

16. A steam treated pelletized feed composition comprising granules according to any of claims 1- 10.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 72649405 P
- EP 1222256 B1
- US 20060193897 A1
- EP 1257631 B1
- US 6852336 B
- US 4689297 A
- US 5324649 A
- EP 656058 B1
- US 454332 A
- US 6248706 B
- EP 804532 B1
- US 6534466 B
- WO 03000625 A
- US 6872696 B
- WO 9951210 A
- WO 0047060 A

**Non-patent literature cited in the description**

- Pelleting Cost Center. **FAIRFIELD, D.** Feed Manufacturing Technology IV. American Feed Industry Association, Arlington, VA, 1994, 110-139
- **ENGELEN AJ ; VAN DER HEEFT FC ; RANDSDORP PH ; SOMERS WA ; SCHAEFER J ; VAN DER VAT BJ.** Determination of phytase activity in feed by a colorimetric enzymatic method: collaborative interlaboratory study. *J AOAC Int.,* May 2001, vol. 84 (3), 629-33
- *Enzymes in Animal Nutrition, Proceedings of the 1st Symposium, Kartause Ittingen, Switzerland,* October 1993